# EUROPEAN PATENT APPLICATION

(11) **EP 2 319 939 A2**
(43) Date of publication of application: **11.05.2011**
(21) Application number: 10182252.6
(22) Date of filing: 23.10.2000
(51) Int. Cl.: C12Q 1/68

(54) **Gene expression profiling of inflammatory bowel disease**

(30) Priority: 21.10.1999 US 160835 P
(62) Divisional of application: 00988479.2
(71) Applicant: CASE WESTERN RESERVE UNIVERSITY, Cleveland, OH 44106-4971 (US)
(72) Inventor: Chakravarti, Shukti, Lutherville, MD 21093 (US)
(74) Representative: Goodfellow, Hugh Robin

(57) **Abstract**

The present invention relates to methods for identifying and/or classifying patients with inflammatory bowel diseases (IBD), particularly patients with Crohn's disease or ulcerative colitis. Gene expression profiling shows broad and fundamental differences in the pathogenic mechanism of UC and CD. The subject method is based on the findings that certain genes are differentially expressed in intestinal tissue of IBD patients compared with related normal cells, such as normal colon cells. That change can be used to identify or classify IBD cells by the upregulation and/or downregulation of expression of particular genes, alterations in protein levels or modification, or changes at the genomic level (such as mutation, methylation, etc), e.g., an event which is implicated in the pathology of inflammatory bowel diseases.

## Description

### Field of the Invention

The present invention provides nucleic acid sequences and proteins encoded thereby, as well as probes derived from the nucleic acid sequences, antibodies directed to the encoded proteins, and diagnostic and prognostic methods for detecting inflammatory bowel diseases, especially Crohn's disease and ulcerative colitis.

### Background of the Invention

Inflammatory bowel disease (IBD) is a common disease of the Western World. Symptoms include chronic intestinal inflammation, diarrhea, bloody stool, weight loss and bowel obstruction. With no obvious cure, surgery is a frequent outcome. Major IBD-subtypes, Ulcerative colitis and Crohn's disease, share similar demographic and epidemiological features with as much as 10% of the cases being clinically indistinguishable. However, key differences in tissue damage and prognosis suggests distinct underlying pathogenic processes. In UC, inflammatory infiltrates and tissue damage is limited to the mucosal layer with extensive disruption of the mucosa, crypt abscesses, neutrophilic infiltrations. While transmural damage, thickening of intestinal wall and increased trichrome staining for connective tissue are typical of Crohn's disease.

IBD is classically viewed as a multi-step disease with two major players. First, initiating events of environmental origin, such as exotoxins, and other microbial factors. Secondly, the responding host immune system that leads to normal healing in unaffected, but inflammation and tissue response in IBD patients. Thus, past IBD studies have focused on selected environmental factors and cytokines, immune cells and inflammatory proteins.

### Summary of the Invention

One aspect of the present invention relates to methods for identifying genes which are up- or down-regulated in intestinal tissue of patients who have, or are at risk of developing, an inflammatory bowel disease or disorder. In general, the method provides for
(i) generating a first library of nucleic acid probes representative of genes expressed by intestinal tissue of an animal without apparent symptoms and/or risk for an inflammatory bowel disease or disorder;
(ii) generating a second library of nucleic acid probes representative of genes expressed by intestinal tissue of an animal which has symptoms of, and/or is at risk for developing, an inflammatory bowel disease or disorder; and
(iii) identifying genes that up-or down-regulated, e.g., by at least a predetermined fold difference, in the second library of nucleic acids relative to the first library of nucleic acids.
The subject method can include such further steps as: cloning those genes which are up- or down-regulated; generating nucleic acid probes for detecting the level of expression of those genes which are up- or down-regulated; and providing kits, such as microarrays, including probes for detecting the level of expression of those genes which are up- or down-regulated.

In one preferred embodiment, the present invention relates to methods of determining the phenotype of a cell, particularly a cell of intestinal origin, comprising detecting the differential expression, relative to a normal cell, of at least one gene (and more preferably 10, 25 or even 50 different genes) shown in Table 1 (herein the "IBD gene set"), or other IBD genes identified according to the subject differential display methodolgy, wherein the assay detects a difference in the level of expression of at least a factor of two, preferably by at least a factor of five, and more preferably by at least a factor of twenty, or at least a factor of fifty. In certain embodiments, a change in the level of expression of at least 10 percent, and more preferably at least 25, 50,75, or 90 percent, of the IBD gene set indicates an increased risk of the patient having, or developing, an inflammatory bowel disease. In preferred embodiments, the changes (up- or down-regulation) of IBD genes which indicate an increased risk of the patient having, or developing, an inflammatory bowel disease are in the same direction, and more preferably of the same approximate magnitude, as set forth in Table 1.

In other embodiments, the assay can be used to detect mutations effecting the chromosomal integrity of an IBD gene, e.g., by detecting mutations (insertions, deletions, point mutations, methylation levels) to the coding sequence or transcriptional regulatory sequences and, e.g., effecting one or more alleles of an IBD gene. In still other embodiments, the method can be used to detect alterations in splicing of IBD transcripts, changes in the levels of IBD proteins, changes in post-translational modification of IBD proteins, and/or changes in half-lives for IBD proteins.

In addition to detecting alterations at the nucleic acid level, the subject method can be carried out by detecting the level of protein encoded by an IBD gene, e.g., by immunoassay or other proteometric technique.

The subject method can be used diagnostically, e.g., to identify patients who have developed, or are at risk of developing, an inflammatory bowel disease. In this regard, the subject method can also be used to distinguish the cause of inflammatory bowel symptoms, e.g., to distinguish between UC and CD. The subject method can also be used prognostically for patients already diagnosed with an IBD, e.g., to determine the aggressive or stage of their disease. In either case, the subject method can be used to augment treatment decisions

The samples used to determine the level of expression of an IBD gene or gene product can include biopsied materials. However, in certain embodiments, genes which are up- or down-regulated in inflammatory bowel diseases encode proteins which can be detected in bodily fluids or in fecal matter. For example, as described in further detail below, certain of the IBD genes encode secreted factors. Accordingly, the present invention specifically contemplates assays which detect a change in the serum level (or other bodily fluid) of one or more secreted IBD gene products. In such embodiments, the method may make use of an immunoassay, e.g., including an antibody panel (or other binding protein) to detect the level of an IBD gene product in the fluid sample.

Another aspect of the present invention provides libraries of nucleic acid probes ("IBD probes") for indexing the level of expression of one or more IBD genes. For instance, such nucleic acid probes can be immobilized on a solid support, e.g., paper, membranes, filters, chips, pins or glass slides, or any other appropriate substrate. In preferred embodiments, the invention provides a microarray of IBD probes for detecting transcripts of at least 5 different IBD genes, more preferably at least 10, and even more preferably at least 25, 50, 75, 100, 125 or all 146 of the IBD gene set described herein.

In general, the subject IBD probes will be isolated nucleic acids (oligonucleotides) comprising a nucleotide sequence which hybridizes under stringent conditions to a sequence of Table 1 or a sequence complementary thereto. In a related embodiment, the nucleic acid is at least about 80% or about 100% identical to a sequence corresponding to at least about 12, at least about 15, at least about 25, or at least about 40 consecutive nucleotides up to the full length of one of the IBD gene set (see Table 1 ) or a sequence complementary thereto or up to the full length of the gene of which said sequence is a fragment. In certain embodiments, a nucleic acid of the present invention includes at least about five, at least about ten, or at least about twenty nucleic acids from a novel coding sequence region of an IBD gene. The IBD probes may include a label group attached thereto and able to be detected. The label group may be selected from radioisotopes, fluorescent compounds, enzymes, and enzyme co-factors.

In certain embodiments, the kit may further include instructions for using the kit, solutions for suspending or fixing the cells, detectable tags or labels, solutions for rendering a nucleic acid susceptible to hybridization, solutions for lysing cells, or solutions for the purification of nucleic acids.

As mentioned above, the subject method also includes kits comprising one or more antibodies ("anti-IBD antibody") immunoreactive with IBD gene products, preferably secreted IBD products or IBD gene products which can be detected in fecal matter. In preferred embodiments, the antibodies can be provided in an array, e.g., in separate wells of a microtitre plate or immobilized on a solid support, e.g., paper, membranes, filters, chips, pins or glass slides, or any other appropriate substrate. The anti-IBD antibodies may include a label group attached thereto and able to be detected. The label group may be selected from radioisotopes, fluorescent compounds, enzymes, and enzyme co-factors. The kit may further include other reagents for detecting the presence of IBD protein:anti-IBD antibody conjugates. In certain embodiments, the kit may further include instructions for using the kit, solutions for suspending or fixing the cells, detectable tags or labels, solutions for rendering a polypeptide susceptible to the binding of an antibody, solutions for lysing cells, or solutions for the purification of polypeptides.

Still another aspect of the present invention provides drug screening assays for identifying agents which can be used to treat or manage the effects of an inflammatory bowel disease or disorder, e.g., by counteracting the effects of the up- or down-regulation of one or more of the subject IBD genes. Such assays include formats which detect agents that inhibit or potentiate expression (transcription or translation) of an IBD gene, formats which detect agents that inhibit or potentiate an activity of an IBD gene product (enzymatic activity, protein-protein interaction, protein-DNA interaction, etc), formats which detect agents that which alter the splicing of IBD gene transcripts, and formats which detect agents that which shorten or extend the half-life of an IBD gene product. For each of the assay embodiments set out above, the assay is preferably repeated for a variegated library of at least 100 different test compounds, though preferably libraries of at least 10^{3,} 10^{5,} 10⁷, and 10⁹ compunds are tested. The test compound can be, for example, peptides, carbohydrates, nucleic acids and other small organic molecules, and/or natural product extracts.

In yet another aspect, the invention provides pharmaceutical compositions including agents, e.g., which have been identied by the assays described herein, which alter the level of expression or splicing of one or more IBD genes, alter the activity or half-life of an IBD gene product, or which alter the post-translational modification of an IBD gene product.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II(D. N. Glover ed., 1985); Oligonucleotide Synthesis (M. J. Gait ed., 1984); Mullis et al. U.S. Patent No. 4,683,195; Nucleic Acid Hybridization (B.D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

### Brief Description of the Figure

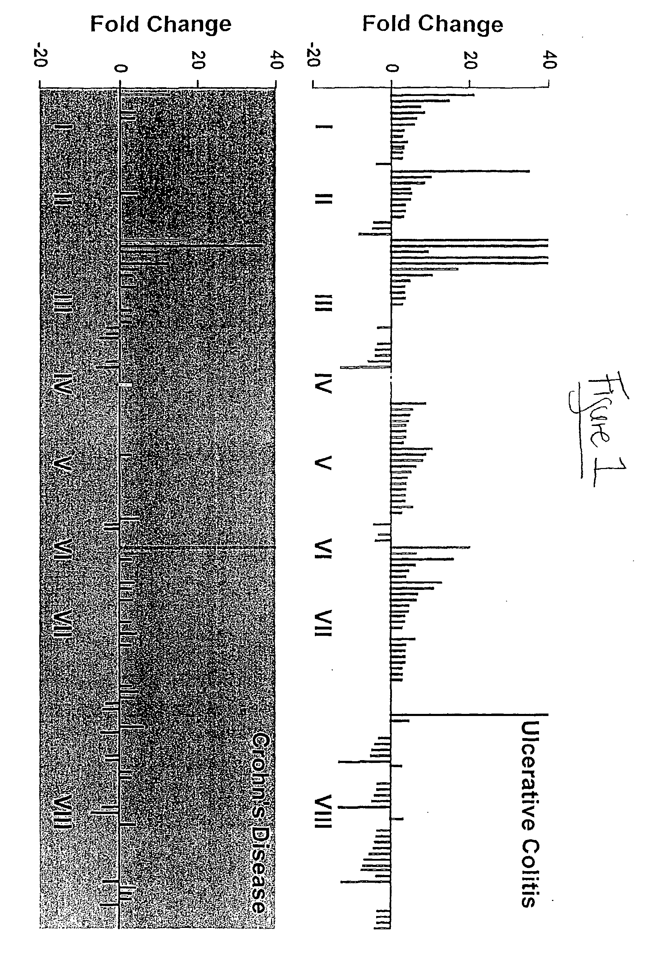

Figure 1 depicts IBD genes which are up- or down-regulated in intestinal cell samples form patients diagonsed with Crohn's disease (CD) of ulcerative colitis (UC).

### Detailed Description of the Invention

### I. General

Inflammatory bowel diseases, such as Crohn's disease (affecting primarily the small intestine) and ulcerative colitis (affecting primarily the large bowel), are chronic diseases of unknown etiology which result in the destruction of the mucosal surface, inflammation, scar and adhesion formation during repair, and significant morbidity to the affected individuals.

This invention relates in part to novel methods for identifying and/or classifying patients with inflammatory bowel diseases (IBD), particularly patients with Crohn's disease or ulcerative colitis. Gene expression profiling, for the first time, shows broad and fundamental differences in the pathogenic mechanism of UC and CD. The subject method is based on the findings that certain genes are differentially expressed in intestinal tissue of IBD patients compared with related normal cells, such as normal colon cells. That change can be used to thereby identify or classify IBD cells by the upregulation and/or downregulation of expression of particular genes, alterations in protein levels or modification, or changes at the genomic level (such as mutation, methylation, etc), e.g., an event which is implicated in the pathology of inflammatory bowel diseases.

Accordingly, in one aspect, the invention also provides biomarkers, such as nucleic acid markers or antobodies, for diagnosing IBD. The invention also provides proteins encoded by these nucleic acid markers.

The invention also features methods for identifying drugs useful for treatment of such disorders. Unlike prior methods, the invention provides a means for identifying IBD patients, and IBD cells at an early stage of development, so that treatment can be determined for early intervention. As described below, certain IBDs are associated with higher risks of cancer, e.g., colon cancer. This allows early detection of potentially cancerous conditions, and treatment of those cancerous conditions prior to spread of the cancerous cells throughout the body, or prior to development of an irreversible cancerous condition.

To obtain a global view of the biological processes gone awry in IBD, the gene expression profiles of UC and CD was elucidated using high-density DNA oligonucleotide microarrays. Six UC and six CD patients, were selected as a source of discarded colon tissues based on the following criteria. Moderate to severe inflammation was confirmed by histology for all twelve patient samples. All samples were taken from colonic tissues. Each disease group of six members was balanced for age and male to female ratio. For controls, discarded colonic tissue from six cancer patients, age and gender- balanced as the IBD patients, were used. Since the IBD tissues came from left or the right colon, half of the control samples were obtained from right and half from the left colon.

In two independent experiments using identical UC RNA, hybridization responses were similar with a correlation coefficient of 0.97, confirming high reproducibility of arrays and experimental conditions.

Gene expression profiles of UC and CD, normalized to control have certain features in common. However, beyond these, the profiles suggest two distinctive disease signatures. Genes showing three-fold or greater changes in expression levels were assigned to seven functional classes as indicated in Table 1. Among these, IBD hallmarks, such as cytokine members of the IL-8 super-family, inflammation marker phospholipase A2, MMPs and collagen type I were elevated, further validating the profiles. A striking upregulation of intestinal paneth cell-specific defensins (DEF5 andDEF6) corroborates past claims of microbial contributionto IBD. Defensins are inducible antimicrobial peptides recognized increasingly as mediators of epithelial host defense. Unlike most upregulated genes showing greater activities in UC than CD, the defensins are far more active in CD. This may be due to a relatively healthier epithelial layer in CD, or an intrinsic difference in presentation of microbial factors between the two diseases.

A majority of the genes in group I belong to the IL-8 superfamily. Produced by T-cells, macrophages, fibroblasts and platelets in response to common mediators of the inflammatory process (TNFa, IFNg and LPS). These are chemoattractants for neutrophils, basophils and other immune-cells, studied in the context of acute and chronic inflammatory diseases have also been cited as upregulated in both UC and CD. The expression profiles, however, show stronger IL-8 activities in UC. Interestingly, the GRO genes, structurally and functionally related to the IL-8 members, are only overexpressed in UC. The GRO proteins, (macrophage inflammatory proteins) are heparin-binding, mitogenic factors associated with melanomas. In group II (inflammation and healing-related), UC and CD are clearly divergent. Of the dozen genes differentially regulated in UC, only one, PLA2, a known inflammation marker is altered in CD as well. Elevated nitricoxide synthase, super oxide dismutase and serum amyloid A messages in UC are part of an acute inflammatory response. Interestingly, metallothioneins, intracellular storage molecules for metal-ions such as zinc (Zn), are markedly down-regulated in UC. Extensive epithelial destruction in UC may be responsible for reduced levels of many epithelial gene products, including metallothioneins. Since zinc enhances epithelial repair in the gut, reduced Zn-storage capabilities may further contribute to tissue destruction.

Two lipocalin genes, HNL and NGAL are 35- and 10-fold upregulated in UC. These lipocalins reportedly bind lipophilic molecules like retinoic acid and bacterial peptides with important growth and immunomodulatory consequences. Of particular relevance to UC is the association of NGAL-overexpression with lung and colon adenocarcinomas. Altered regulation of four cancer-related genes in UC, further strengthens its ties to colon cancer. DD96, upregulated by 4.8 fold in UC, is a gene with low activities in normal epithelium but overexpressed in lung, breast and colon carcinoma. Furthermore, both MXI1and DRA are down-regulated in UC. MXII, a negative regulator of MYC is a potential tumor suppressor. DRA, an epithelial anion transporter is normally present in the gastrointestinal mucosa and its absence is associated with proliferative and neoplastic transformation of the crypt epithelium. Increased incidence of colon cancer in UC patients is well known. One or more of the cancer-related genes identified in the UC profile may be contributing to the neoplastic propensity in UC.

Group III (cell proliferation/regulation/transcription factor) genes show considerable overlap in UC and CD expression patterns; 43 % of the differentially regulated genes are common to both diseases. A surprising finding was extremely high upregulation of the REG1B and the REG1A (lithostathine) genes in UC (155. and 75 fold) and CD (17 and 36 fold). The islet regeneration genes code for pancreatic stone or thread proteins. In normal pancreas these proteins may bind to and prevent precipitation of calcium carbonate and serve as islet-cell-specific growth factors. Their overexpression after pancreatectomy or acute pancreatitis, ectopic expression in colon and rectal cancer suggest a role in cell dedifferentiation and proliferation. In IBD, REGs may specifically induce cell proliferation at sites of inflammation. With a similar role, PAP is another member of this gene family also overexpressed in both diseases, and associated with carcinomas of the liver, pancreas and intestine. In vitro PAP induced extensive bacterial aggregation and an antibacterial role was suggested. Although entirely speculative, it is possible that the three REG members in IBD not only mark inflammation, but are specifically induced by some microbial factors and contribute to the antimicrobial-defense system. Two genes for S100 calcium-binding myeloid-related proteins are up-regulated, possibly involved in monocyte-macrophage differentiation during inflammation. These have been hypothesized to mark a subpopulation of activated macrophages in UC. Calgranulin B (MRP14) is also elevated in psoriatic skin. A third S100 gene (calgizzarin) up-regulated in UC was placed in the cancer-related group for its clear connection to carcinomas. NF-kappa B reportedly up-regulated in UC and CD was only three-fold up-regulated in the CD expression profile. The implications of down-regulated cell cycle-regulators and transcription factors, such as ZNF9 and transcription factor IIIa in UC, liver-specific leucine zipper protein in CD and sorcin, a calcium -binding, multi-drug resistance protein in both are unclear.

The group that shows the most dramatic difference in UC and CD is V (HLA and immune function-related). Twenty- two of the twenty -five genes (88%) in this category are differently regulated in UC, as opposed to four (16%) in CD. We found elevated transcripts for seven HLA class II antigens including HLA DPB1, HLA-DRB1 and DQ. These results support past genetic studies that have connected specific class II HLA alleles, with UC in defined populations. A majority of the other members of this group in UC are immunoglobulins associated with B cell development and antibody production. This is the most compelling evidence for a strong immune-function component in UC that is clearly not there in CD.

Extracellular matrix and its remodeling, required for adhesion, infiltration and proliferation of inflammatory cells, has become a recent focus in IBD studies. Starting from the superficial mucous barrier, changes in mucins were considered to compromise barrier-integrity against exogenous antigens. Disruptions of basement membranes underlying vascular endothelial cells were proposed to allow recuitment of circulating inflammatory cells and interstitial ECM changes to foster inflammation and healing-related activities. Expression profiling allowed a broad look at all of these components. Of the twenty-seven genes in group VII (ecm, remodeling, cytoskeletal and mucins), expression of twenty-one and twelve are altered in UC and CD, respectively. Only six of these are common to both diseases. MMP 12 or human metalloelastase, not connected to IBD thus far, was most up-regulated in UC and CD. Secreted by macrophages, MMP12 has been studied in the context of macrophage-mediated proteolysis and matrix invasion in lung inflammation and emphesyma. In addition to degrading elastin it is active on a range of substrates including fibrinogen, plasminogen, laminin and proteoglycans. Interestingly, elastase inhibitor (elafin) is up-regulated in both diseases, possibly to limit MMP 12 activity. Cigarette smoke and emphesyma-studies have noted increased elastinolytic activities in lung macrophages and a resulting elastase-elastase inhibitor imbalance considered to favor emphesyma. Since MMP12 is far more up-regulated in UC (16 fold) than CD (3fold), an intriguing possibility is that the beneficial effects of cigarette-smoking in UC may be due to the same elastase-elafin imbalance, in this case, contributing to anti-angiogenic and clotting favoring conditions. In agreement with recent studies MMP 1, 3 and 9 were markedly up-regulated in UC. MMP 1 is an interstitial collagenase while MMP3 and 9 have a broad range substrate including basement membrane type IV collagens. Interstitial ECM collagen messages COL1A1 and COL1A2, were elevated in both diseases, while COL3AI (collagen type III) and basement membrane COL4A2 were differentially up-regulated in UC. However, robust MMP activities may allow for their rapid turnover in UC. Comparatively lower MMP levels in CD may lead to increased deposition as noted by several studies. Messages for Collagen type VI, a microfibril forming cell adhesive collagen, were 4-6 fold elevated in UC and may be important in platelet cell adhesion during inflammation. Additional fundamental differences were noted in the expression pattern of this group in UC and CD.

The study yielded an unprecedented view of a repertoire of transcripts regulated differently in UC and CD over control samples.

### II.Definitions

For convenience, the meaning of certain terms and phrases used in the specification, examples, and appended claims, are provided below.

The term "an aberrant expression", as applied to a nucleic acid of the present invention, refers to level of expression of that nucleic acid which differs from the level of expression of that nucleic acid in healthy tissue, or which differs from the activity of the polypeptide present in a healthy subject. An activity of a polypeptide can be aberrant because it is stronger than the activity of its native counterpart. Alternatively, an activity can be aberrant because it is weaker or absent relative to the activity of its native counterpart. An aberrant activity can also be a change in the activity; for example, an aberrant polypeptide can interact with a different target peptide. A cell can have an aberrant expression level of a gene due to overexpression or underexpression of that gene.

The term "agonist", as used herein, is meant to refer to an agent that mimics or upregulates (e.g., potentiates or supplements) the bioactivity of a protein, e.g., an IBD protein. An agonist can be a wild-type protein or derivative thereof having at least one bioactivity of the wild-type protein. An agonist can also be a compound that upregulates expression of a gene or which increases at least one bioactivity of a protein. An agonist can also be a compound which increases the interaction of a polypeptide with another molecule, e.g., a target peptide or nucleic acid.

The term "allele", which is used interchangeably herein with "allelic variant", refers to alternative forms of a gene or portions thereof. Alleles occupy the same locus or position on homologous chromosomes. When a subject has two identical alleles of a gene, the subject is said to be homozygous for that gene or allele. When a subject has two different alleles of a gene, the subject is said to be heterozygous for the gene. Alleles of a specific gene can differ from each other in a single nucleotide, or several nucleotides, and can include substitutions, deletions, and/or insertions of nucleotides. An allele of a gene can also be a form of a gene containing mutations.

The term "allelic variant of a polymorphic region of a gene" refers to a region of a gene having one of several nucleotide sequences found in that region of the gene in other individuals.

"Altered" nucleic acid sequences encoding an IBD gene product as used herein include those with deletions, insertions, or substitutions of different nucleotides resulting in a polynucleotide that encodes the same or a functionally equivalent IBD gene product. Included within this definition are polymorphisms which may or may not be readily detectable using a particular oligonucleotide probe of the polynucleotide encoding an IBD gene product, and improper or unexpected hybridization to alleles, with a locus other than the normal chromosomal locus for the polynucleotide sequence encoding an IBD gene product. The encoded protein may also be "altered" and contain deletions, insertions, or substitutions of amino acid residues which produce a silent change and result in a functionally equivalent IBD gene product. Deliberate amino acid substitutions may be made on the basis of similarity in polarity, charge, solubility, hydrophobicity, hydrophilicity, and/or the amphipathic nature of the residues as long as the biological or immunological activity of an IBD gene product is retained. For example, negatively charged amino acids may include aspartic acid and glutamic acid; positively charged amino acids may include lysine and arginine; and amino acids with uncharged polar head groups having similar hydrophilicity values may include leucine, isoleucine, and valine, glycine and alanine, asparagine and glutamine, serine and threonine, and phenylalanine and tyrosine.

"Amino acid sequence" as used herein refers to an oligopeptide, peptide, polypeptide, or protein sequence, and fragment thereof, and to naturally occurring or synthetic molecules. Fragments of an IBD gene product are preferably about 5 to about 15 amino acids in length and retain the biological activity or the immunological activity of an IBD gene product. Where "amino acid sequence" is recited herein to refer to an amino acid sequence of a naturally occurring protein molecule, amino acid sequence, and like terms, are not meant to limit the amino acid sequence to the complete, native amino acid sequence associated with the recited protein molecule.

"Antagonist" as used herein is meant to refer to an agent that downregulates (e.g., suppresses or inhibits) at least one bioactivity of a protein. An antagonist can be a compound which inhibits or decreases the interaction between a protein and another molecule, e.g., a target peptide or enzyme substrate. An antagonist can also be a compound that downregulates expression of a gene or which reduces the amount of expressed protein present.

"Amplification" as used herein refers to the production of additional copies of a nucleic acid sequence and is generally carried out using polymerase chain reaction (PCR) technologies well known in the art (Dieffenbach, C. W. and G. S. Dveksler (1995) PCR Primer, a Laboratory Manual, Cold Spring Harbor Press, Plainview, N.Y.).

The term "antibody" as used herein is intended to include whole antibodies, e.g., of any isotype (IgG, IgA, IgM, IgE, etc), and includes fragments thereof which are also specifically reactive with a vertebrate, e.g., mammalian, protein. Antibodies can be fragmented using conventional techniques and the fragments screened for utility in the same manner as described above for whole antibodies. Thus, the term includes segments of proteolytically-cleaved or recombinantly-prepared portions of an antibody molecule that are capable of selectively reacting with a certain protein. Nonlimiting examples of such proteolytic and/or recombinant fragments include Fab, F(ab')2, Fab' , Fv, and single chain antibodies (scFv) containing a V[L] and/or V[H] domain joined by a peptide linker. The scFv's may be covalently or non-covalently linked to form antibodies having two or more binding sites. The subject invention includes polyclonal, monoclonal, or other purified preparations of antibodies and recombinant antibodies.

A disease, disorder, or condition "associated with" or "characterized by" an aberrant expression of an IBD nucleic acid refers to a disease, disorder, or condition in a subject which is caused by, contributed to by, or causative of an aberrant level of expression of a nucleic acid.

"Biological activity" or "bioactivity" or "activity" or "biological function", which are used interchangeably, herein mean an effector or antigenic function that is directly or indirectly performed by a polypeptide (whether in its native or denatured conformation), or by any subsequence thereof. Biological activities include binding to polypeptides, binding to other proteins or molecules, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The term "biomarker" refers a biological molecule, e.g., a nucleic acid, peptide, hormone, etc., whose presence or concentration can be detected and correlated with a known condition, such as a disease state.

"Cells," "host cells", or "recombinant host cells" are terms used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

The terms "complementary" or "complementarity", as used herein, refer to the natural binding of polynucleotides under permissive salt and temperature conditions by base-pairing. For example, the sequence "A-G-T" binds to the complementary sequence "T-C-A". Complementarity between two single-stranded molecules may be "partial", in which only some of the nucleic acids bind, or it may be complete when total complementarity exists between the single stranded molecules. The degree of complementarity between nucleic acid strands has significant effects on the efficiency and strength of hybridization between nucleic acid strands. This is of particular importance in amplification reactions, which depend upon binding between nucleic acids strands and in the design and use of PNA molecules.

A "composition comprising a given polynucleotide sequence" as used herein refers broadly to any composition containing the given polynucleotide sequence. The composition may comprise a dry formulation or an aqueous solution. Compositions comprising polynucleotide sequences encoding an IBD gene product or fragments thereof may be employed as hybridization probes. The probes may be stored in freeze-dried form and may be associated with a stabilizing agent such as a carbohydrate. In hybridizations, the probe may be deployed in an aqueous solution containing salts (e.g., NaCl), detergents (e.g., SDS) and other components (e.g., Denhardt's solution, dry milk, salmon sperm DNA, etc.).

"Consensus", as used herein, refers to a nucleic acid sequence which has been resequenced to resolve uncalled bases, has been extended using XL-PCR (Perkin Elmer, Norwalk, Conn.) in the 5' and/or the 3' direction and resequenced, or has been assembled from the overlapping sequences of more than one Incyte Clone using a computer program for fragment assembly (e.g., GELVIEW fragment assembly system, GCG, Madison, Wis.). Some sequences have been both extended and assembled to produce the consensus sequence.

The term "correlates with expression of a polynucleotide", as used herein, indicates that the detection of the presence of ribonucleic acid that is similar to one of IBD genes by northern analysis is indicative of the presence of mRNA encoding an IBD gene product in a sample and thereby correlates with expression of the transcript from the polynucleotide encoding the protein.

A "deletion", as used herein, refers to a change in the amino acid or nucleotide sequence and results in the absence of one or more amino acid residues or nucleotides.

As is well known, genes or a particular polypeptide may exist in single or multiple copies within the genome of an individual. Such duplicate genes may be identical or may have certain modifications, including nucleotide substitutions, additions or deletions, which all still code for polypeptides having substantially the same activity. The term "DNA sequence encoding an IBD polypeptide" may thus refer to one or more genes within a particular individual. Moreover, certain differences in nucleotide sequences may exist between individual organisms, which are called alleles. Such allelic differences may or may not result in differences in amino acid sequence of the encoded polypeptide yet still encode a polypeptide with the same biological activity.

The term "equivalent" is understood to include nucleotide sequences encoding functionally equivalent polypeptides. Equivalent nucleotide sequences will include sequences that differ by one or more nucleotide substitutions, additions or deletions, such as allelic variants; and will, therefore, include sequences that differ from the nucleotide sequence of the nucleic acids referred to in Table I due to the degeneracy of the genetic code.

As used herein, the terms "gene", "recombinant gene", and "gene construct" refer to a nucleic acid of the present invention associated with an open reading frame, including both exon and (optionally) intron sequences.

A "recombinant gene" refers to nucleic acid encoding a polypeptide and comprising exon sequences, though it may optionally include intron sequences which are derived from, for example, a related or unrelated chromosomal gene. The term "intron" refers to a DNA sequence present in a given gene which is not translated into protein and is generally found between exons.

The term "growth" or "growth state" of a cell refers to the proliferative state of a cell as well as to its differentiative state. Accordingly, the term refers to the phase of the cell cycle in which the cell is, e.g., G0, G1, G2, prophase, metaphase, or telophase, as well as to its state of differentiation, e.g., undifferentiated, partially differentiated, or fully differentiated. Without wanting to be limited, differentiation of a cell is usually accompanied by a decrease in the proliferative rate of a cell.

"Homology" or "identity" or "similarity" refers to sequence similarity between two peptides or between two nucleic acid molecules, with identity being a more strict comparison. Homology and identity can each be determined by comparing a position in each sequence which may be aligned for purposes of comparison. When a position in the compared sequence is occupied by the same base or amino acid, then the molecules are identical at that position. A degree of homology or similarity or identity between nucleic acid sequences is a function of the number of identical or matching nucleotides at positions shared by the nucleic acid sequences. A degree of identity of amino acid sequences is a function of the number of identical amino acids at positions shared by the amino acid sequences. A degree of homology or similarity of amino acid sequences is a function of the number of amino acids, i.e., structurally related, at positions shared by the amino acid sequences. An "unrelated" or "non-homologous" sequence shares less than 40% identity, though preferably less than 25% identity, with one of the sequences of the present invention.

The term "percent identical" refers to sequence identity between two amino acid sequences or between two nucleotide sequences. Identity can each be determined by comparing a position in each sequence which may be aligned for purposes of comparison. When an equivalent position in the compared sequences is occupied by the same base or amino acid, then the molecules are identical at that position; when the equivalent site occupied by the same or a similar amino acid residue (e.g., similar in steric and/or electronic nature), then the molecules can be referred to as homologous (similar) at that position. Expression as a percentage of homology, similarity, or identity refers to a function of the number of identical or similar amino acids at positions shared by the compared sequences. Various alignment algorithms and/or programs may be used, including FASTA, BLAST, or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, Md. In one embodiment, the percent identity of two sequences can be determined by the GCG program with a gap weight of 1, e.g., each amino acid gap is weighted as if it were a single amino acid or nucleotide mismatch between the two sequences.

Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, California, USA. Preferably, an alignment program that permits gaps in the sequence is utilized to align the sequences. The Smith-Waterman is one type of algorithm that permits gaps in sequence alignments. See Meth. Mol. Biol. 70: 173-187 (1997). Also, the GAP program using the Needleman and Wunsch alignment method can be utilized to align sequences. An alternative search strategy uses MPSRCH software, which runs on a MASPAR computer. MPSRCH uses a Smith-Waterman algorithm to score sequences on a massively parallel computer. This approach improves ability to pick up distantly related matches, and is especially tolerant of small gaps and nucleotide sequence errors. Nucleic acid-encoded amino acid sequences can be used to search both protein and DNA databases.

Databases with individual sequences are described in Methods in Enzymology, ed. Doolittle, *supra.* Databases include Genbank, EMBL, and DNA Database of Japan (DDBJ).

The term "hybridization" , as used herein, refers to any process by which a strand of nucleic acid binds with a complementary strand through base pairing.

An "insertion" or "addition", as used herein, refers to a change in an amino acid or nucleotide sequence resulting in the addition of one or more amino acid residues or nucleotides, respectively, as compared to the naturally occurring molecule.

The term "interact" as used herein is meant to include detectable interactions (e.g., biochemical interactions) between molecules, such as interaction between protein-protein, protein-nucleic acid, nucleic acid-nucleic acid, and protein-small molecule or nucleic acid-small molecule in nature.

The term "isolated" as used herein with respect to nucleic acids, such as DNA or RNA, refers to molecules separated from other DNA_{S}, or RNAs, respectively, that are present in the natural source of the macromolecule. The term isolated as used herein also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

"Microarray" refers to an array of distinct polynucleotides or oligonucleotides synthesized on a substrate, such as paper, nylon or other type of membrane, filter, chip, glass slide, or any other suitable solid support.

The terms "modulated" and "differentially regulated" as used herein refer to both upregulation (i.e., activation or stimulation (e.g., by agonizing or potentiating)) and downregulation (i.e., inhibition or suppression (e.g., by antagonizing, decreasing or inhibiting)).

The term "mutated gene" refers to an allelic form of a gene, which is capable of altering the phenotype of a subject having the mutated gene relative to a subject which does not have the mutated gene. If a subject must be homozygous for this mutation to have an altered phenotype, the mutation is said to be recessive. If one copy of the mutated gene is sufficient to alter the genotype of the subject, the mutation is said to be dominant. If a subject has one copy of the mutated gene and has a phenotype that is intermediate between that of a homozygous and that of a heterozygous subject (for that gene), the mutation is said to be co-dominant.

As used herein, the term "nucleic acid" refers to polynucleotides such as deoxyribonucleic acid (DNA), and, where appropriate, ribonucleic acid (RNA). The term should also be understood to include, as equivalents, analogs of either RNA or DNA made from nucleotide analogs, and, as applicable to the embodiment being described, single (sense or antisense) and double-stranded polynucleotides. ESTs, chromosomes, cDNAs, mRNAs, and rRNAs are representative examples of molecules that may be referred to as nucleic acids.

The term "nucleotide sequence complementary to the nucleotide sequence of Table 1" refers to the nucleotide sequence of the complementary strand of a nucleic acid strand having designated in the GenBank accession referred to in Table 1. The term "complementary strand" is used herein interchangeably with the term "complement". The complement of a nucleic acid strand can be the complement of a coding strand or the complement of a non-coding strand.

The term "polymorphism" refers to the coexistence of more than one form of a gene or portion (e.g., allelic variant) thereof. A portion of a gene of which there are at least two different forms, i.e., two different nucleotide sequences, is referred to as a "polymorphic region of a gene". A polymorphic region can be a single nucleotide, the identity of which differs in different alleles. A polymorphic region can also be several nucleotides long.

A "polymorphic gene" refers to a gene having at least one polymorphic region.

As used herein, the term "promoter" means a DNA sequence that regulates expression of a selected DNA sequence operably linked to the promoter, and which effects expression of the selected DNA sequence in cells. The term encompasses "tissue specific" promoters, i.e., promoters which effect expression of the selected DNA sequence only in specific cells (e.g., cells of a specific tissue). The term also covers so-called "leaky" promoters, which regulate expression of a selected DNA primarily in one tissue, but cause expression in other tissues as well. The term also encompasses non-tissue specific promoters and promoters that constitutively expressed or that are inducible (i.e., expression levels can be controlled).

The terms "protein", "polypeptide", and "peptide" are used interchangeably herein when referring to a gene product.

The term "sample", as used herein, is used in its broadest sense. A biological sample suspected of containing nucleic acid encoding an IBD gene product, or fragments thereof, or an IBD gene product itself may comprise a bodily fluid, extract from a cell, chromosome, organelle, or membrane isolated from a cell, a cell, genomic DNA, RNA, or cDNA (in solution or bound to a solid support, a tissue, a tissue print, and the like).

"Small molecule" as used herein, is meant to refer to a composition, which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

As used herein, the term "specifically hybridizes" or "specifically detects" refers to the ability of a nucleic acid molecule of the invention to hybridize to at least a portion of, for example, approximately 6, 12, 15, 20, 30, 50, 100, 150, 200, 300, 350, 400, 500, 750, or 1000 contiguous nucleotides of a nucleic acid designated in any one of SEQ ID Nos: 1-146, or a sequence complementary thereto, or naturally occurring mutants thereof, such that it has less than 15%, preferably less than 10%, and more preferably less than 5% background hybridization to a cellular nucleic acid (e.g., mRNA or genomic DNA) encoding a different protein. In preferred embodiments, the oligonucleotide probe detects only a specific nucleic acid, e.g., it does not substantially hybridize to similar or related nucleic acids, or complements thereof.

A "substitution", as used herein, refers to the replacement of one or more amino acids or nucleotides by different amino acids or nucleotides, respectively.

"Transcriptional regulatory sequence" is a generic term used throughout the specification to refer to DNA sequences, such as initiation signals, enhancers, and promoters, which induce or control transcription of protein coding sequences with which they are operably linked. In preferred embodiments, transcription of one of the genes is under the control of a promoter sequence (or other transcriptional regulatory sequence) which controls the expression of the recombinant gene in a cell-type in which expression is intended. It will also be understood that the recombinant gene can be under the control of transcriptional regulatory sequences which are the same or which are different from those sequences which control transcription of the naturally-occurring forms of the polypeptide.

As used herein, the term "transgene" means a nucleic acid sequence (or an antisense transcript thereto) which has been introduced into a cell. A transgene could be partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can also be present in a cell in the form of an episome. A transgene can include one or more transcriptional regulatory sequences and any other nucleic acid, such as introns, that may be necessary for optimal expression of a selected nucleic acid.

A "transgenic animal" refers to any animal, preferably a non-human mammal, bird or an amphibian, in which one or more of the cells of the animal contain heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or *in vitro* fertilization, but rather is directed to the introduction of a recombinant DNA molecule. This molecule may be integrated within a chromosome, or it may be extra-chromosomally replicating DNA. In the typical transgenic animals described herein, the transgene causes cells to express a recombinant form of one of the subject polypeptide, e.g. either agonistic or antagonistic forms. However, transgenic animals in which the recombinant gene is silent are also contemplated, as for example, the FLP or CRE recombinase dependent constructs described below. Moreover, "transgenic animal" also includes those recombinant animals in which gene disruption of one or more genes is caused by human intervention, including both recombination and antisense techniques.

The term "treating" as used herein is intended to encompass curing as well as ameliorating at least one symptom of the condition or disease.

The term "wild-type allele" refers to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes.

### III. Nucleic Acids of the Present Invention

As described below, one aspect of the invention pertains to isolated nucleic acids, variants, and/or equivalents of such nucleic acids.

Nucleic acids of the present invention have been identified as differentially expressed in IBD cells, e.g., UC- or CD-derived cell lines (relative to the expression levels in normal tissue, e.g., normal colon tissue and/or normal non-colon tissue), such as Table 1. In certain embodiments, the subject nucleic acids are differentially expressed by at least a factor of two, preferably at least a factor of five, even more preferably at least a factor of twenty, still more preferably at least a factor of fifty.

Table 1 indicates those sequences which are over- or underexpressed in a CD- or UC-derived cells relative to normal tissue.

Genes which are upregulated, such as oncogenes or mitogens, or downregulated, such as tumor suppressors, in IBD cells may be targets for diagnostic or therapeutic techniques.

Preferred nucleic acids of the present invention encode a polypeptide comprising at least a portion of a polypeptide encoded by one of Table 1, or can hybridize to the coding sequences thereof. For example, preferred nucleic acid molecules for use as probes/primers or antisense molecules (i.e., noncoding nucleic acid molecules) can comprise at least about 12, 20, 30, 50, 60, 70, 80, 90, or 100 base pairs in length up to the length of the complete gene. Coding nucleic acid molecules can comprise, for example, from about 50, 60, 70, 80, 90, or 100 base pairs up to the length of the complete gene.

Another aspect of the invention provides a nucleic acid which hybridizes under low, medium, or high stringency conditions to a nucleic acid sequence represented by one of Table 1, or a sequence complementary thereto. Appropriate stringency conditions which promote DNA hybridization, for example, 6.0 x sodium chloride/sodium citrate (SSC) at about 45 °C, followed by a wash of 2.0 x SSC at 50 °C, are known to those skilled in the art or can be found in Current Protocols in Molecular Biology, John Wiley & Sons, N.Y. (1989), 6.3.1-12.3.6. For example, the salt concentration in the wash step can be selected from a low stringency of about 2.0 x SSC at 50 °C to a high stringency of about 0.2 x SSC at 50 °C. In addition, the temperature in the wash step can be increased from low stringency conditions at room temperature, about 22 °C, to high stringency conditions at about 65 °C. Both temperature and salt may be varied, or temperature or salt concentration may be held constant while the other variable is changed. In a preferred embodiment, a nucleic acid of the present invention will hybridize to one of Table 1, or a sequence complementary thereto, under moderately stringent conditions, for example at about 2.0 x SSC and about 40 °C. In a particularly preferred embodiment, a nucleic acid of the present invention will hybridize to one of Table 1, or a sequence complementary thereto, under high stringency conditions.

In one embodiment, the invention provides nucleic acids which hybridize under low stringency conditions of 6 x SSC at room temperature followed by a wash at 2 x SSC at room temperature.

In another embodiment, the invention provides nucleic acids which hybridize under high stringency conditions of 2 x SSC at 65 °C followed by a wash at 0.2 x SSC at 65 °C.

Nucleic acids having a sequence that differs from the nucleotide sequences shown in one of Table 1, or a sequence complementary thereto, due to degeneracy in the genetic code, are also within the scope of the invention. Such nucleic acids encode functionally equivalent peptides (i.e., a peptide having equivalent or similar biological activity) but differ in sequence from the sequence shown in the sequence listing due to degeneracy in the genetic code. For example, a number of amino acids are designated by more than one triplet. Codons that specify the same amino acid, or synonyms (for example, CAU and CAC each encode histidine) may result in "silent" mutations which do not affect the amino acid sequence of a polypeptide. However, it is expected that DNA sequence polymorphisms that do lead to changes in the amino acid sequences of the subject polypeptides will exist among mammals. One skilled in the art will appreciate that these variations in one or more nucleotides (e.g., up to about 3-5% of the nucleotides) of the nucleic acids encoding polypeptides having an activity of a polypeptide may exist among individuals of a given species due to natural allelic variation.

Also within the scope of the invention are nucleic acids encoding splicing variants of proteins encoded by a nucleic acid of Table 1, or a sequence complementary thereto, or natural homologs of such proteins. Such homologs can be cloned by hybridization or PCR, as further described herein.

Techniques for producing and probing nucleic acid sequence libraries are described, for example, in Sambrook et al., "Molecular Cloning: A Laboratory Manual" (New York, Cold Spring Harbor Laboratory, 1989). The cDNA can be prepared by using primers based on a sequence from Table 1. In one embodiment, the cDNA library can be made from only poly-adenylated mRNA. Thus, poly-T primers can be used to prepare cDNA from the mRNA. Alignment of Table 1 can result in identification of a related polypeptide or polynucleotide. Some of the polynucleotides disclosed herein contains repetitive regions that were subject to masking during the search procedures. The information about the repetitive regions is discussed below.

Constructs of polynucleotides having sequences of Table 1 can be generated synthetically. Alternatively, single-step assembly of a gene and entire plasmid from large numbers of oligodeoxyribonucleotides is described by Stemmer et al., Gene (Amsterdam) (1995) 164(l):49-53*.* In this method, assembly PCR (the synthesis of long DNA sequences from large numbers of oligodeoxyribonucleotides (oligos)) is described. The method is derived from DNA shuffling (Stemmer, Nature (1994) 370:389-391), and does not rely on DNA ligase, but instead relies on DNA polymerase to build increasingly longer DNA fragments during the assembly process. For example, a 1.1-kb fragment containing the TEM-1 beta-lactamase-encoding gene (bla) can be assembled in a single reaction from a total of 56 oligos, each 40 nucleotide (nt) in length. The synthetic gene can be PCR amplified and cloned in a vector containing the tetracycline-resistance gene (Tc-R) as the sole selectable marker. Without relying on ampicillin (Ap) selection, 76% of the Tc-R colonies were Ap-R, making this approach a general method for the rapid and cost-effective synthesis of any gene.

The IBD probes of the present invention can be useful because they provide a method for detecting mutations in wild-type IBD genes of the present invention. Nucleic acid probes which are complementary to a wild-type gene of the present invention and can form mismatches with mutant genes are provided, allowing for detection by enzymatic or chemical cleavage or by shifts in electrophoretic mobility.

Likewise, probes based on the subject sequences can be used to detect the level of transcripts of IBD genes, for use, for example, in prognostic or diagnostic assays. In preferred embodiments, the probe further comprises a label group attached thereto and able to be detected, e.g., the label group is selected from radioisotopes, fluorescent compounds, chemiluminescent compounds, enzymes, and enzyme co-factors.

Full-length cDNA molecules comprising the disclosed nucleic acids are obtained as follows. A subject nucleic acid or a portion thereof comprising at least about 12, 15, 18, or 20 nucleotides up to the full length of a sequence represented in Table 1, preferably Table 1, or a sequence complementary thereto, may be used as a hybridization probe to detect hybridizing members of a cDNA library using probe design methods, cloning methods, and clone selection techniques as described in U.S. Patent No. 5,654,173, "Secreted Proteins and Polynucleotides Encoding Them," incorporated herein by reference. Libraries of cDNA may be made from selected tissues, such as normal or tumor tissue, or from tissues of a mammal treated with, for example, a pharmaceutical agent. Preferably, the tissue is the same as that used to generate the nucleic acids, as both the nucleic acid and the cDNA represent expressed genes. Most preferably, the cDNA library is made from the biological material described herein in the Examples. Alternatively, many cDNA libraries are available commercially. (Sambrook et al., Molecular Cloning: A Laboratory Manual, 2nd Ed. (Cold Spring Harbor Press, Cold Spring Harbor, NY 1989). The choice of cell type for library construction may be made after the identity of the protein encoded by the nucleic acid-related gene is known. This will indicate which tissue and cell types are likely to express the related gene, thereby containing the mRNA for generating the cDNA.

Members of the library that are larger than the nucleic acid, and preferably that contain the whole sequence of the native message, may be obtained. To confirm that the entire cDNA has been obtained, RNA protection experiments may be performed as follows. Hybridization of a full-length cDNA to an mRNA may protect the RNA from RNase degradation. If the cDNA is not full length, then the portions of the mRNA that are not hybridized may be subject to RNase degradation. This may be assayed, as is known in the art, by changes in electrophoretic mobility on polyacrylamide gels, or by detection of released monoribonucleotides. Sambrook et al., Molecular Cloning: A Laboratory Manual, 2nd Ed. (Cold Spring Harbor Press, Cold Spring Harbor, NY 1989). In order to obtain additional sequences 5' to the end of a partial cDNA, 5' RACE (PCR Protocols: A Guide to Methods and Applications (Academic Press, Inc. 1990)) may be performed.

Genomic DNA may be isolated using nucleic acids in a manner similar to the isolation of full-length cDNAs. Briefly, the nucleic acids, or portions thereof, may be used as probes to libraries of genomic DNA. Preferably, the library is obtained from the cell type that was used to generate the nucleic acids. Most preferably, the genomic DNA is obtained from the biological material described herein in the Example. Such libraries may be in vectors suitable for carrying large segments of a genome, such as P1 or YAC, as described in detail in Sambrook *et al.,* 9.4-9.30. In addition, genomic sequences can be isolated from human BAC libraries, which are commercially available from Research Genetics, Inc., Huntville, Alabama, USA, for example. In order to obtain additional 5' or 3' sequences, chromosome walking may be performed, as described in *Sambrook et al.,* such that adjacent and overlapping fragments of genomic DNA are isolated. These may be mapped and pieced together, as is known in the art, using restriction digestion enzymes and DNA ligase.

Using the nucleic acids of the invention, corresponding full length genes can be isolated using both classical and PCR methods to construct and probe cDNA libraries. Using either method, Northern blots, preferably, may be performed on a number of cell types to determine which cell lines express the gene of interest at the highest rate.

Classical methods of constructing cDNA libraries are taught in Sambrook et al., supra. With these methods, cDNA can be produced from mRNA and inserted into viral or expression vectors. Typically, libraries of mRNA comprising poly(A) tails can be produced with poly(T) primers. Similarly, cDNA libraries can be produced using the instant sequences as primers.

PCR methods may be used to amplify the members of a cDNA library that comprise the desired insert. In this case, the desired insert may contain sequence from the full length cDNA that corresponds to the instant nucleic acids. Such PCR methods include gene trapping and RACE methods.

"Rapid amplification of cDNA ends," or RACE, is a PCR method of amplifying cDNAs from a number of different RNAs. The cDNAs may be ligated to an oligonucleotide linker and amplified by PCR using two primers. One primer may be based on sequence from the instant nucleic acids, for which full length sequence is desired, and a second primer may comprise a sequence that hybridizes to the oligonucleotide linker to amplify the cDNA. A description of this method is reported in PCT Pub. No. WO 97/19110.

In preferred embodiments of RACE, a common primer may be designed to anneal to an arbitrary adaptor sequence ligated to cDNA ends (Apte and Siebert, Biotechniques 15:890-893, 1993; Edwards et al., Nuc. Acids Res. 19:5227-5232, 1991). When a single gene-specific RACE primer is paired with the common primer, preferential amplification of sequences between the single gene specific primer and the common primer occurs. Commercial cDNA pools modified for use in RACE are available.

Another PCR-based method generates full-length cDNA library with anchored ends without specific knowledge of the cDNA sequence. The method uses lock-docking primers (I-VI), where one primer, poly TV (I-III) locks over the polyA tail of eukaryotic mRNA producing first strand synthesis and a second primer, polyGH (IV-VI) locks onto the polyC tail added by terminal deoxynucleotidyl transferase (TdT). This method is described in PCT Pub. No. WO 96/40998.

The promoter region of a gene generally is located 5' to the initiation site for RNA polymerase II. Hundreds of promoter regions contain the "TATA" box, a sequence such as TATTA or TATAA, which is sensitive to mutations. The promoter region can be obtained by performing 5' RACE using a primer from the coding region of the gene. Alternatively, the cDNA can be used as a probe for the genomic sequence, and the region 5' to the coding region is identified by "walking up."

If the gene is highly expressed or differentially expressed, the promoter from the gene may be of use in a regulatory construct for a heterologous gene.

Once the full-length cDNA or gene is obtained, DNA encoding variants can be prepared by site-directed mutagenesis, described in detail in Sambrook *et al.,* 15.3-15.63. The choice of codon or nucleotide to be replaced can be based on the disclosure herein on optional changes in amino acids to achieve altered protein structure and/or function.

As an alternative method to obtaining DNA or RNA from a biological material, nucleic acid comprising nucleotides having the sequence of one or more nucleic acids of the invention can be synthesized. Thus, the invention encompasses nucleic acid molecules ranging in length from 12 nucleotides (corresponding to at least 12 contiguous nucleotides which hybridize under stringent conditions to or are at least 80% identical to a nucleic acid represented by one of Table I, or a sequence complementary thereto) up to a maximum length suitable for one or more biological manipulations, including replication and expression, of the nucleic acid molecule. The invention includes but is not limited to (a) nucleic acid having the size of a full gene, and comprising at least one of Table 1, or a sequence complementary thereto; (b) the nucleic acid of (a) also comprising at least one additional gene, operably linked to permit expression of a fusion protein; (c) an expression vector comprising (a) or (b); (d) a plasmid comprising (a) or (b); and (e) a recombinant viral particle comprising (a) or (b). Construction of (a) can be accomplished as described below in part IV.

The sequence of a nucleic acid of the present invention is not limited and can be any sequence of A, T, G, and/or C (for DNA) and A, U, G, and/or C (for RNA) or modified bases thereof, including inosine and pseudouridine. The choice of sequence will depend on the desired function and can be dictated by coding regions desired, the intron-like regions desired, and the regulatory regions desired.

### IV. Identification of Functional and Structural Motifs of Novel Genes Using Art-Recognized Methods

Translations of the nucleotide sequence of the nucleic acids, cDNAs, or full genes can be aligned with individual known sequences. Similarity with individual sequences can be used to determine the activity of the polypeptides encoded by the polynucleotides of the invention. For example, sequences that show similarity with a chemokine sequence may exhibit chemokine activities. Also, sequences exhibiting similarity with more than one individual sequence may exhibit activities that are characteristic of either or both individual sequences.

The full length sequences and fragments of the polynucleotide sequences of the nearest neighbors can be used as probes and primers to identify and isolate the full length sequence of the nucleic acid. The nearest neighbors can indicate a tissue or cell type to be used to construct a library for the full-length sequences of the nucleic acid.

Typically, the nucleic acids are translated in all six frames to determine the best alignment with the individual sequences. The sequences disclosed herein in the Sequence Listing are in a 5' to 3' orientation and translation in three frames can be sufficient (with a few specific exceptions as described in the Examples). These amino acid sequences are referred to, generally, as query sequences, which will be aligned with the individual sequences.

Nucleic acid sequences can be compared with known genes by any of the methods disclosed above. Results of individual and query sequence alignments can be divided into three categories: high similarity, weak similarity, and no similarity. Individual alignment results ranging from high similarity to weak similarity provide a basis for determining polypeptide activity and/or structure.

Parameters for categorizing individual results include: percentage of the alignment region length where the strongest alignment is found, percent sequence identity, and p value.

The percentage of the alignment region length is calculated by counting the number of residues of the individual sequence found in the region of strongest alignment. This number is divided by the total residue length of the query sequence to find a percentage.

Percent sequence identity is calculated by counting the number of amino acid matches between the query and individual sequence and dividing total number of matches by the number of residues of the individual sequence found in the region of strongest alignment.

P value is the probability that the alignment was produced by chance. For a single alignment, the p value can be calculated according to Karlin et al., Proc. Natl. Acad. Sci. 87: 2264 (1990) and Karlin et al., Proc. Natl. Acad. Sci. 90: (1993). The p value of multiple alignments using the same query sequence can be calculated using an heuristic approach described in Altschul et al., Nat. Genet. 6: 119 (1994). Alignment programs such as BLAST program can calculate the p value.

The boundaries of the region where the sequences align can be determined according to Doolittle, Methods in Enzymology, *supra;* BLAST or FASTA programs; or by determining the area where the sequence identity is highest.

Another factor to consider for determining identity or similarity is the location of the similarity or identity. Strong local alignment can indicate similarity even if the length of alignment is short. Sequence identity scattered throughout the length of the query sequence also can indicate a similarity between the query and profile sequences.

### A. High Similarity

For the alignment results to be considered high similarity, the percent of the alignment region length, typically, is at least about 55% of total length query sequence; more typically, at least about 58%; even more typically; at least about 60% of the total residue length of the query sequence. Usually, percent length of the alignment region can be as much as about 62%; more usually, as much as about 64%; even more usually, as much as about 66%.

Further, for high similarity, the region of alignment, typically, exhibits at least about 75% of sequence identity; more typically, at least about 78%; even more typically; at least about 80% sequence identity. Usually, percent sequence identity can be as much as about 82%; more usually, as much as about 84%; even more usually, as much as about 86%.

The p value is used in conjunction with these methods. If high similarity is found, the query sequence is considered to have high similarity with a profile sequence when the p value is less than or equal to about 10⁻²; more usually; less than or equal to about 10⁻³; even more usually; less than or equal to about 10⁻⁴. More typically, the p value is no more than about 10⁻⁵; more typically; no more than or equal to about 10⁻¹⁰; even more typically; no more than or equal to about 10⁻¹⁵ for the query sequence to be considered high similarity.

### B. Weak Similarity

For the alignment results to be considered weak similarity, there is no minimum percent length of the alignment region nor minimum length of alignment. A better showing of weak similarity is considered when the region of alignment is, typically, at least about 15 amino acid residues in length; more typically, at least about 20; even more typically; at least about 25 amino acid residues in length. Usually, length of the alignment region can be as much as about 30 amino acid residues; more usually, as much as about 40; even more usually, as much as about 60 amino acid residues.

Further, for weak similarity, the region of alignment, typically, exhibits at least about 35% of sequence identity; more typically, at least about 40%; even more typically; at least about 45% sequence identity. Usually, percent sequence identity can be as much as about 50%; more usually, as much as about 55%; even more usually, as much as about 60%.

If low similarity is found, the query sequence is considered to have weak similarity with a profile sequence when the p value is usually less than or equal to about 10⁻²; more usually; less than or equal to about 10⁻³; even more usually; less than or equal to about 10⁻⁴. More typically, the p value is no more than about 10⁻⁵; more usually; no more than or equal to about 10⁻¹⁰; even more usually; no more than or equal to about 10⁻¹⁵ for the query sequence to be considered weak similarity.

### C. Similarity Determined by Sequence Identity

Sequence identity alone can be used to determine similarity of a query sequence to an individual sequence and can indicate the activity of the sequence. Such an alignment, preferably, permits gaps to align sequences. Typically, the query sequence is related to the profile sequence if the sequence identity over the entire query sequence is at least about 15%; more typically, at least about 20%; even more typically, at least about 25%; even more typically, at least about 50%. Sequence identity alone as a measure of similarity is most useful when the query sequence is usually, at least 80 residues in length; more usually, 90 residues; even more usually, at least 95 amino acid residues in length. More typically, similarity can be concluded based on sequence identity alone when the query sequence is preferably 100 residues in length; more preferably, 120 residues in length; even more preferably, 150 amino acid residues in length.

### D. Determining Activity from Alignments with Profile and Multiple Aligned Sequences

Translations of the nucleic acids can be aligned with amino acid profiles that define either protein families or common motifs. Also, translations of the nucleic acids can be aligned to multiple sequence alignments (MSA) comprising the polypeptide sequences of members of protein families or motifs. Similarity or identity with profile sequences or MSAs can be used to determine the activity of the polypeptides encoded by nucleic acids or corresponding cDNA or genes. For example, sequences that show an identity or similarity with a chemokine profile or MSA can exhibit chemokine activities.

Profiles can designed manually by (1) creating a MSA, which is an alignment of the amino acid sequence of members that belong to the family and (2) constructing a statistical representation of the alignment. Such methods are described, for example, in Birney et al., Nucl. Acid Res. 24(14): 2730-2739 (1996).

MSAs of some protein families and motifs are publicly available. For example, these include MSAs of 547 different families and motifs. These MSAs are described also in Sonnhammer et al., Proteins 28: 405-420 (1997). Other sources are also available in the world wide web. A brief description of these MSAs is reported in Pascarella et al., Prot. Eng. 9(3): 249-251 (1996).

Techniques for building profiles from MSAs are described in Sonnhammer *et al., supra;* Birney *et al., supra;* and Methods in Enzymology, vol. 266: "Computer Methods for Macromolecular Sequence Analysis," 1996, ed. Doolittle, Academic Press, Inc., a division of Harcourt Brace & Co., San Diego, California, USA.

Similarity between a query sequence and a protein family or motif can be determined by (a) comparing the query sequence against the profile and/or (b) aligning the query sequence with the members of the family or motif.

Typically, a program such as Searchwise can be used to compare the query sequence to the statistical representation of the multiple alignment, also known as a profile. The program is described in Birney *et al., supra.* Other techniques to compare the sequence and profile are described in Sonnhammer *et al., supra* and Doolittle, *supra.*

Next, methods described by Feng et al., J. Mol. Evol. 25: 351-360 (1987) and Higgins et al., CABIOS 5: 151-153 (I989) can be used align the query sequence with the members of a family or motif, also known as a MSA. Computer programs, such as PILI;UP, can be used. See *Feng et al., infra.*

The following factors are used to determine if a similarity between a query sequence and a profile or MSA exists: (1) number of conserved residues found in the query sequence, (2) percentage of conserved residues found in the query sequence, (3) number of frameshifts, and (4) spacing between conserved residues.

Some alignment programs that both translate and align sequences can make any number of frameshifts when translating the nucleotide sequence to produce the best alignment. The fewer frameshifts needed to produce an alignment, the stronger the similarity or identity between the query and profile or MSAs. For example, a weak similarity resulting from no frameshifts can be a better indication of activity or structure of a query sequence, than a strong similarity resulting from two frameshifts. Preferably, three or fewer frameshifts are found in an alignment; more preferably two or fewer frameshifts; even more preferably, one or fewer frameshifts; even more preferably, no frameshifts are found in an alignment of query and profile or MSAs.

Conserved residues are those amino acids that are found at a particular position in all or some of the family or motif members. For example, most known chemokines contain four conserved cysteines. Alternatively, a position is considered conserved if only a certain class of amino acids is found in a particular position in all or some of the family members. For example, the N-terminal position may contain a positively charged amino acid, such as lysine, arginine, or histidine.

Typically, a residue of a polypeptide is conserved when a class of amino acids or a single amino acid is found at a particular position in at least about 40% of all class members; more typically, at least about 50%; even more typically, at least about 60% of the members. Usually, a residue is conserved when a class or single amino acid is found in at least about 70% of the members of a family or motif; more usually, at least about 80%; even more usually, at least about 90%; even more usually, at least about 95%.

A residue is considered conserved when three unrelated amino acids are found at a particular position in the some or all of the members; more usually, two unrelated amino acids. These residues are conserved when the unrelated amino acids are found at particular positions in at least about 40% of all class member; more typically, at least about 50%; even more typically, at least about 60% of the members. Usually, a residue is conserved when a class or single amino acid is found in at least about 70% of the members of a family or motif; more usually, at least about 80%; even more usually, at least about 90%; even more usually, at least about 95%.

A query sequence has similarity to a profile or MSA when the query sequence comprises at least about 25% of the conserved residues of the profile or MSA; more usually, at least about 30%; even more usually; at least about 40%. Typically, the query sequence has a stronger similarity to a profile sequence or MSA when the query sequence comprises at least about 45% of the conserved residues of the profile or MSA; more typically, at least about 50%; even more typically; at least about 55%.

### V. Therapeutic Nucleic Acid Constructs

One aspect of the invention relates to the use of the isolated nucleic acid, e.g., Table 1, or a sequence complementary thereto, in antisense therapy. As used herein, antisense therapy refers to administration or *in situ* generation of oligonucleotide molecules or their derivatives which specifically hybridize (e.g., bind) under cellular conditions with the cellular mRNA and/or genomic DNA, thereby inhibiting transcription and/or translation of that gene. The binding may be by conventional base pair complementarity, or, for example, in the case of binding to DNA duplexes, through specific interactions in the major groove of the double helix. In general, antisense therapy refers to the range of techniques generally employed in the art, and includes any therapy which relies on specific binding to oligonucleotide sequences.

An antisense construct of the present invention can be delivered, for example, as an expression plasmid which, when transcribed in the cell, produces RNA which is complementary to at least a unique portion of the cellular mRNA. Alternatively, the antisense construct is an oligonucleotide probe which is generated *ex vivo* and which, when introduced into the cell, causes inhibition of expression by hybridizing with the mRNA and/or genomic sequences of a subject nucleic acid. Such oligonucleotide probes are preferably modified oligonucleotides which are resistant to endogenous nucleases, e.g., exonucleases and/or endonucleases, and are therefore stable *in vivo.* Exemplary nucleic acid molecules for use as antisense oligonucleotides are phosphoramidate, phosphorothioate and methylphosphonate analogs of DNA (see also U.S. Patents 5,176,996; 5,264,564; and 5,256,775). Additionally, general approaches to constructing oligomers useful in antisense therapy have been reviewed, for example, by Van der Krol et al. (1988) BioTechniques 6:958-976; and Stein et al. (1988) Cancer Res 48:2659-2668. With respect to antisense DNA, oligodeoxyribonucleotides derived from the translation initiation site, e.g., between the -10 and +10 regions of the nucleotide sequence of interest, are preferred.

Antisense approaches involve the design of oligonucleotides (either DNA or RNA) that are complementary to mRNA. The antisense oligonucleotides will bind to the mRNA transcripts and prevent translation. Absolute complementarity, although preferred, is not required. In the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

Oligonucleotides that are complementary to the 5' end of the mRNA, e.g., the 5' untranslated sequence up to and including the AUG initiation codon, should work most efficiently at inhibiting translation. However, sequences complementary to the 3' untranslated sequences of mRNAs have recently been shown to be effective at inhibiting translation of mRNAs as well. (Wagner, R. 1994. Nature 372:333). Therefore, oligonucleotides complementary to either the 5' or 3' untranslated, non-coding regions of a gene could be used in an antisense approach to inhibit translation of endogenous mRNA. Oligonucleotides complementary to the 5' untranslated region of the mRNA should include the complement of the AUG start codon. Antisense oligonucleotides complementary to mRNA coding regions are typically less efficient inhibitors of translation but could also be used in accordance with the invention. Whether designed to hybridize to the 5', 3', or coding region of subject mRNA, antisense nucleic acids should be at least six nucleotides in length, and are preferably less that about 100 and more preferably less than about 50, 25, 17 or 10 nucleotides in length.

Regardless of the choice of target sequence, it is preferred that *in vitro* studies are first performed to quantitate the ability of the antisense oligonucleotide to quantitate the ability of the antisense oligonucleotide to inhibit gene expression. It is preferred that these studies utilize controls that distinguish between antisense gene inhibition and nonspecific biological effects of oligonucleotides. It is also preferred that these studies compare levels of the target RNA or protein with that of an internal control RNA or protein. Additionally, it is envisioned that results obtained using the antisense oligonucleotide are compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

The oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (e.g., for targeting host cell receptors), or agents facilitating transport across the cell membrane (see, e.g., Letsinger et al., 1989, Proc. Natl. Acad. Sci. U.S.A. 86:6553-6556; Lemaitre et al., 1987, Proc. Natl. Acad. Sci. 84:648-652; PCT Publication No. WO 88/09810, published December 15, 1988) or the blood-brain barrier (see, e.g., PCT Publication No. WO 89/10134, published April 25, 1988), hybridization-triggered cleavage agents (See, e.g., Krol et al., 1988, BioTechniques 6:958-976), or intercalating agents (See, e.g., Zon, 1988, Pharm. Res. 5:539-549). To this end, the oligonucleotide may be conjugated to another molecule, e.g., a peptide, hybridization triggered cross-linking agent, transport agent, hybridization-triggered cleavage agent, etc.

The antisense oligonucleotide may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxytriethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including but not limited to arabinose, 2-fluoroarabinose, xylulose, and hexose.

The antisense oligonucleotide can also contain a neutral peptide-like backbone. Such molecules are termed peptide nucleic acid (PNA)-oligomers and are described, e.g., in Perry- O'Keefe et al. (1996) Proc. Natl. Acad. Sci. U.S.A. 93:14670 and in Eglom et al. (1993) Nature 365:566. One advantage of PNA oligomers is their capability to bind to complementary DNA essentially independently from the ionic strength of the medium due to the neutral backbone of the DNA. In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet a further embodiment, the antisense oligonucleotide is an α-anomeric oligonucleotide. An a-anomeric oligonucleotide forms specific double-stranded hybrids with complementary RNA in which, contrary to the usual β-units, the strands run parallel to each other (Gautier et al., 1987, Nucl. Acids Res. 15:6625-6641). The oligonucleotide is a 2'-O-methylribonucleotide (Inoue et al., 1987, Nucl. Acids Res. 15:6131-12148), or a chimeric RNA-DNA analogue (Inoue et al., 1987, FEBS Lett. 215:327-330).

Oligonucleotides of the invention may be synthesized by standard methods known in the art, e.g., by use of an automated DNA synthesizer (such as are commercially available from Biosearch, Applied Biosystems, etc.). As examples, phosphorothioate oligonucleotides may be synthesized by the method of Stein et al. (1988, Nucl. Acids Res. 16:3209), methylphosphonate olgonucleotides can be prepared by use of controlled pore glass polymer supports (Sarin et al., 1988, Proc. Natl. Acad. Sci. U.S.A. 85:7448-7451), etc.

While antisense nucleotides complementary to a coding region sequence can be used, those complementary to the transcribed untranslated region and to the region comprising the initiating methionine are most preferred.

The antisense molecules can be delivered to cells which express the target nucleic acid *in vivo.* A number of methods have been developed for delivering antisense DNA or RNA to cells; e.g., antisense molecules can be injected directly into the tissue site, or modified antisense molecules, designed to target the desired cells (e.g., antisense linked to peptides or antibodies that specifically bind receptors or antigens expressed on the target cell surface) can be administered systemically.

However, it is often difficult to achieve intracellular concentrations of the antisense sufficient to suppress translation on endogenous mRNAs. Therefore, a preferred approach utilizes a recombinant DNA construct in which the antisense oligonucleotide is placed under the control of a strong pol III or pol II promoter. The use of such a construct to transfect target cells in the patient will result in the transcription of sufficient amounts of single stranded RNAs that will form complementary base pairs with the endogenous transcripts and thereby prevent translation of the target mRNA. For example, a vector can be introduced in *vivo* such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art for replication and expression in mammalian cells. Expression of the sequence encoding the antisense RNA can be by any promoter known in the art to act in mammalian, preferably human cells. Such promoters can be inducible or constitutive. Such promoters include but are not limited to: the SV40 early promoter region (Bemoist and Chambon, 198l, Nature 290:304-310), the promoter contained in the 3' long terminal repeat of Rous sarcoma virus (Yamamoto et al., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al, 1982, Nature 296:39-42), etc. Any type of plasmid, cosmid, YAC or viral vector can be used to prepare the recombinant DNA construct which can be introduced directly into the tissue site; e.g., the choroid plexus or hypothalamus. Alternatively, viral vectors can be used which selectively infect the desired tissue (e.g., for brain, herpesvirus vectors may be used), in which case administration may be accomplished by another route (e.g., systemically).

In another aspect of the invention, ribozyme molecules designed to catalytically cleave target mRNA transcripts can be used to prevent translation of target mRNA and expression of a target protein (See, e.g., PCT International Publication WO90/11364, published October 4, 1990; Sarver et al., 1990, Science 247:1222-1225 and U.S. Patent No. 5,093,246). While ribozymes that cleave mRNA at site specific recognition sequences can be used to destroy target mRNAs, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach, 1988, Nature, 334:585-591. Preferably the ribozyme is engineered so that the cleavage recognition site is located near the 5' end of the target mRNA; i.e., to increase efficiency and minimize the intracellular accumulation of non-functional mRNA transcripts.

The ribozymes of the present invention also include RNA endoribonucleases (hereinafter "Cech-type ribozymes") such as the one which occurs naturally in *Tetrahymena thermophila* (known as the IVS, or L-19 IVS RNA) and which has been extensively described by Thomas Cech and collaborators (Zaug, et al., 1984, Science, 224:574-578; Zaug and Cech, 1986, Science, 231:470-475; Zaug, et al., 1986, Nature, 324:429-433; published International patent application No. WO88/04300 by University Patents Inc.; Been and Cech, 1986, Cell, 47:207-216). The Cech-type ribozymes have an eight base pair active site which hybridizes to a target RNA sequence whereafter cleavage of the target RNA takes place. The invention encompasses those Cech-type ribozymes which target eight base-pair active site sequences that are present in a target gene.

As in the antisense approach, the ribozymes can be composed of modified oligonucleotides (e.g., for improved stability, targeting, etc.) and should be delivered to cells which express the target gene in *vivo.* A preferred method of delivery involves using a DNA construct "encoding" the ribozyme under the control of a strong constitutive pol III or pol II promoter, so that transfected cells will produce sufficient quantities of the ribozyme to destroy endogenous messages and inhibit translation. Because ribozymes, unlike antisense molecules, are catalytic, a lower intracellular concentration is required for efficiency.

Antisense RNA, DNA, and ribozyme molecules of the invention may be prepared by any method known in the art for the synthesis of DNA and RNA molecules. These include techniques for chemically synthesizing oligodeoxyribonucleotides and oligoribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Moreover, various well-known modifications to nucleic acid molecules may be introduced as a means of increasing intracellular stability and half-life. Possible modifications include but are not limited to the addition of flanking sequences of ribonucleotides or deoxyribonucleotides to the 5' and/or 3' ends of the molecule or the use of phosphorothioate or 2^{,} O-methyl rather than phosphodiesterase linkages within the oligodeoxyribonucleotide backbone.

### VI. Polypeptides of the Present Invention

The present invention makes available isolated polypeptides which are isolated from, or otherwise substantially free of other cellular proteins, especially other signal transduction factors and/or transcription factors which may normally be associated with the polypeptide. Subject polypeptides of the present invention include polypeptides encoded by the nucleic acids of Table 1. Polypeptides of the present invention include those proteins which are differentially regulated in IBD tissue, especially colon UC- and CD-derived cell lines (relative to normal cells, e.g., normal colon tissue).

The term "substantially free of other cellular proteins" (also referred to herein as "contaminating proteins") or "substantially pure or purified preparations" are defined as encompassing preparations of polypeptides having less than about 20% (by dry weight) contaminating protein, and preferably having less than about 5% contaminating protein. Functional forms of the subject polypeptides can be prepared, for the first time, as purified preparations by using a cloned nucleic acid as described herein. Full length proteins or fragments corresponding to one or more particular motifs and/or domains or to arbitrary sizes, for example, at least about 5, 10, 25, 50, 75, or 100 amino acids in length are within the scope of the present invention.

For example, isolated polypeptides can be encoded by all or a portion of a nucleic acid sequence shown in any of Table 1, or a sequence complementary thereto. Isolated peptidyl portions of proteins can be obtained by screening peptides recombinantly produced from the corresponding fragment of the nucleic acid encoding such peptides. In addition, fragments can be chemically synthesized using techniques known in the art such as conventional Merrifield solid phase f-Moc or t-Boc chemistry. For example, a polypeptide of the present invention may be arbitrarily divided into fragments of desired length with no overlap of the fragments, or preferably divided into overlapping fragments of a desired length. The fragments can be produced (recombinantly or by chemical synthesis) and tested to identify those peptidyl fragments which can function as either agonists or antagonists of a wild-type (e.g., "authentic") protein.

Another aspect of the present invention concerns recombinant forms of the subject proteins. Recombinant polypeptides preferred by the present invention, in addition to native proteins, as described above are encoded by a nucleic acid, which is at least 60%, more preferably at least 80%, and more preferably 85%, and more preferably 90%, and more preferably 95% identical to an amino acid sequence encoded by Table 1. Polypeptides which are encoded by a nucleic acid that is at least about 98-99% identical with the sequence of Table 1 are also within the scope of the invention. Also included in the present invention are peptide fragments comprising at least a portion of such a protein.

In a preferred embodiment, a polypeptide of the present invention is a mammalian polypeptide and even more preferably a human polypeptide. In particularly preferred embodiment, the polypeptide retains wild-type bioactivity. It will be understood that certain post-translational modifications, e.g., phosphorylation and the like, can increase the apparent molecular weight of the polypeptide relative to the unmodified polypeptide chain.

In another embodiment, the coding sequences for the polypeptide can be incorporated as a part of a fusion gene including a nucleotide sequence encoding a different polypeptide. This type of expression system can be useful under conditions where it is desirable to produce an immunogenic fragment of a polypeptide (see, for example, EP Publication No: 0259149; and Evans et al. (1989) Nature 339:385; Huang et al. (1988) J. Virol. 62:3855; and Schlienger et al. (1992) J. Virol. 66:2). In addition to utilizing fusion proteins to enhance immunogenicity, it is widely appreciated that fusion proteins can also facilitate the expression of proteins, and, accordingly, can be used in the expression of the polypeptides of the present invention (see, for example, Current Protocols in Molecular Biology, eds. Ausubel et al. (N.Y.: John Wiley & Sons, 1991)). In another embodiment, a fusion gene coding for a purification leader sequence, such as a poly-(His)/enterokinase cleavage site sequence at the N-terminus of the desired portion of the recombinant protein, can allow purification of the expressed fusion protein by affinity chromatography using a Ni ²⁺ metal resin. The purification leader sequence can then be subsequently removed by treatment with enterokinase to provide the purified protein (e.g., see Hochuli et al. (1987) J. Chromatography 411:177; and Janknecht et al. PNAS 88:8972).

Techniques for making fusion genes are known to those skilled in the art. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of nucleic acid fragments can be carried out using anchor primers which give rise to complementary overhangs between two consecutive nucleic acid fragments which can subsequently be annealed to generate a chimeric nucleic acid sequence (see, for example, Current Protocols in Molecular Biology, eds. Ausubel et al. John Wiley & Sons: 1992).

The present invention further pertains to methods of producing the subject polypeptides. For example, a host cell transfected with a nucleic acid vector directing expression of a nucleotide sequence encoding the subject polypeptides can be cultured under appropriate conditions to allow expression of the peptide to occur. Suitable media for cell culture are well known in the art. The recombinant polypeptide can be isolated from cell culture medium, host cells, or both using techniques known in the art for purifying proteins including ion-exchange chromatography, gel filtration chromatography, ultrafiltration, electrophoresis, and immunoaffinity purification with antibodies specific for such peptide. In a preferred embodiment, the recombinant polypeptide is a fusion protein containing a domain which facilitates its purification, such as GST fusion protein.

### VII Determining the Function of the Encoded Expression Products

Ribozymes, antisense constructs, dominant negative mutants, and triplex formation can be used to determine function of the expression product of an nucleic acid-related gene.

### A. Ribozymes

Trans-cleaving catalytic RNAs (ribozymes) are RNA molecules possessing endoribonuclease activity. Ribozymes are specifically designed for a particular target, and the target message must contain a specific nucleotide sequence. They are engineered to cleave any RNA species site-specifically in the background of cellular RNA. The cleavage event renders the mRNA unstable and prevents protein expression. Importantly, ribozymes can be used to inhibit expression of a gene of unknown function for the purpose of determining its function in an in vitro or in vivo context, by detecting the phenotypic effect.

One commonly used ribozyme motif is the hammerhead, for which the substrate sequence requirements are minimal. Design of the hammerhead ribozyme is disclosed in Usman et al., Current Opin. Struct. Biol. (1996) 6:527-533. Usman also discusses the therapeutic uses of ribozymes. Ribozymes can also be prepared and used as described in Long et al., FASEB J (1993) 7:25; Symons, Ann. Rev. Biochem. (1992) 61:641; Perrotta et al., Biochem. (1992) 31:16-17; Ojwang et al., Proc. Natl. Acad. Sci. (USA) (1992) 89:10802-10806; and U.S. Patent No. 5,254,678. Ribozyme cleavage of HN-I RNA is described in U.S. Patent No. 5,144,019; methods of cleaving RNA using ribozymes is described in U.S. Patent No. 5,116,742; and methods for increasing the specificity of ribozymes are described in U.S. Patent No. 5,225,337 and Koizumi et al., Nucleic Acid Res. (1989) 17:7059-7071. Preparation and use of ribozyme fragments in a hammerhead structure are also described by Koizumi et al., Nucleic Acids Res. (1989) 17:7059-7071. Preparation and use of ribozyme fragments in a hairpin structure are described by Chowrira and Burke, Nucleic Acids Res. (1992) 20:2835. Ribozymes can also be made by rolling transcription as described in Daubendiek and Kool, Nat. Biotechnol. (1997) 15(3):273-277*.*

The hybridizing region of the ribozyme may be modified or may be prepared as a branched structure as described in Horn and Urdea, Nucleic Acids Res. (1989) 17:6959-67. The basic structure of the ribozymes may also be chemically altered in ways familiar to those skilled in the art, and chemically synthesized ribozymes can be administered as synthetic oligonucleotide derivatives modified by monomeric units. In a therapeutic context, liposome mediated delivery of ribozymes improves cellular uptake, as described in Birikh et al., Eur. J. Biochem. (1997) 245:1-16.

Using the nucleic acid sequences of the invention and methods known in the art, ribozymes are designed to specifically bind and cut the corresponding mRNA species. Ribozymes thus provide a means to inhibit the expression of any of the proteins encoded by the disclosed nucleic acids or their full-length genes. The full-length gene need not be known in order to design and use specific inhibitory ribozymes. In the case of a nucleic acid or cDNA of unknown function, ribozymes corresponding to that nucleotide sequence can be tested in vitro for efficacy in cleaving the target transcript. Those ribozymes that effect cleavage in vitro are further tested in vivo. The ribozyme can also be used to generate an animal model for a disease, as described in Birikh et al., Eur. J Biochem. (1997) 245:1-16. An effective ribozyme is used to determine the function of the gene of interest by blocking its transcription and detecting a change in the cell. Where the gene is found to be a mediator in a disease, an effective ribozyme is designed and delivered in a gene therapy for blocking transcription and expression of the gene.

Therapeutic and functional genomic applications of ribozymes proceed beginning with knowledge of a portion of the coding sequence of the gene to be inhibited. Thus, for many genes, a partial nucleic acid sequence provides adequate sequence for constructing an effective ribozyme. A target cleavage site is selected in the target sequence, and a ribozyme is constructed based on the 5' and 3' nucleotide sequences that flank the cleavage site-Retroviral vectors are engineered to express monomeric and multimeric hammerhead ribozymes targeting the mRNA of the target coding sequence. These monomeric and multimeric ribozymes are tested in vitro for an ability to cleave the target mRNA. A cell line is stably transduced with the retroviral vectors expressing the ribozymes, and the transduction is confirmed by Northern blot analysis and reverse-transcription polymerase chain reaction (RT-PCR). The cells are screened for inactivation of the target mRNA by such indicators as reduction of expression of disease markers or reduction of the gene product of the target mRNA.

### B. Antisense

Antisense nucleic acids are designed to specifically bind to RNA, resulting in the formation of RNA-DNA or RNA-RNA hybrids, with an arrest of DNA replication, reverse transcription or messenger RNA translation. Antisense polynucleotides based on a selected nucleic acid sequence can interfere with expression of the corresponding gene. Antisense polynucleotides are typically generated within the cell by expression from antisense constructs that contain the antisense nucleic acid strand as the transcribed strand. Antisense nucleic acids will bind and/or interfere with the translation of nucleic acid-related mRNA. The expression products of control cells and cells treated with the antisense construct are compared to detect the protein product of the gene corresponding to the nucleic acid. The protein is isolated and identified using routine biochemical methods.

One rationale for using antisense methods to determine the function of the gene corresponding to a nucleic acid is the biological activity of antisense therapeutics. Antisense therapy for a variety of cancers is in clinical phase and has been discussed extensively in the literature. Reed reviewed antisense therapy directed at the Bcl-2 gene in tumors; gene transfer-mediated overexpression of Bcl-2 in tumor cell lines conferred resistance to many types of cancer drugs. (Reed, J.C., N.C.I (1997) 89:988-990). The potential for clinical development of antisense inhibitors of *ras* is discussed by Cowsert, L.M., Anti-Cancer Drug Design (1997) 12:359-371. Additional important antisense targets include leukemia (Geurtz, A.M., Anti-Cancer Drug Design (1997) 12:341-358); human C-ref kinase (Monia, B.P., Anti-Cancer Drug Design (1997) 12:327-339); and protein kinase C (McGraw et al., Anti-Cancer Drug Design (1997) 12:315-326.

Given the extensive background literature and clinical experience in antisense therapy, one skilled in the art can use selected nucleic acids of the invention as additional potential therapeutics. The choice of nucleic acid can be narrowed by first testing them for binding to "hot spot" regions of the genome of cancerous cells. If a nucleic acid is identified as binding to a "hot spot", testing the nucleic acid as an antisense compound in the corresponding cancer cells clearly is warranted.

Ogunbiyi et al., Gastroenterology (1997) 113(3):761-766 describe prognostic use of allelic loss in colon cancer; Barks et al., Genes, Chromosomes, and Cancer (1997) 19(4):278-285 describe increased chromosome copy number detected by FISH in malignant melanoma; Nishizake et al., Genes, Chromosomes, and Cancer (1997) 19(4):267-272 describe genetic alterations in primary breast cancer and their metastases and direct comparison using modified comparative genome hybridization; and Elo et al., Cancer Research (1997) 57(16):3356-3359 disclose that loss of heterozygosity at l6z24.1-q24.2 is significantly associated with metastatic and aggressive behavior of prostate cancer.

### C. Dominant Negative Mutations

As an alternative method for identifying function of the nucleic acid-related gene, dominant negative mutations are readily generated for corresponding proteins that are active as homomultimers. A mutant polypeptide will interact with wild-type polypeptides (made from the other allele) and form a non-functional multimer. Thus, a mutation is in a substrate-binding domain, a catalytic domain, or a cellular localization domain. Preferably, the mutant polypeptide will be overproduced. Point mutations are made that have such an effect. In addition, fusion of different polypeptides of various lengths to the terminus of a protein can yield dominant negative mutants. General strategies are available for making dominant negative mutants. See Herskowitz, Nature (1987) 329:219-222. Such a technique can be used for creating a loss-of function mutation, which is useful for determining the function of a protein.

### D. Triplex Formation

Endogenous gene expression can also be reduced by inactivating or "knocking out" the gene or its promoter using targeted homologous recombination. (E.g., see Smithies et al., 1985, Nature 31?:230-234; Thomas & Capecchi, 1987, Cell 51:503-512; Thompson et al., 1989 Cell 5:313-321; each of which is incorporated by reference herein in its entirety). For example, a mutant, non-functional gene (or a completely unrelated DNA sequence) flanked by DNA homologous to the endogenous gene (either the coding regions or regulatory regions of the gene) can be used, with or without a selectable marker and/or a negative selectable marker, to transfect cells that express that gene *in vivo.* Insertion of the DNA construct, via targeted homologous recombination, results in inactivation of the gene.

Alternatively, endogenous gene expression can be reduced by targeting deoxyribonucleotide sequences complementary to the regulatory region of the target gene (i.e., the gene promoter and/or enhancers) to form triple helical structures that prevent transcription of the gene in target cells in the body. (See generally, Helene, C. 1991, Anticancer Drug Des., 6(6):569-84; Helene, C., et al., 1992, Ann, N.Y. Accad. Sci., 660:27-36; and Maher, L.J., 1992, Bioassays 14(12):807-15).

Nucleic acid molecules to be used in triple helix formation for the inhibition of transcription are preferably single stranded and composed of deoxyribonucleotides. The base composition of these oligonucleotides should promote triple helix formation via Hoogsteen base-pairing rules, which generally require sizable stretches of either purines or pyrimidines to be present on one strand of a duplex. Nucleotide sequences may be pyrimidine-based, which will result in TAT and CGC triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, containing a stretch of G residues. These molecules will form a triple helix with a DNA duplex that is rich in GC pairs, in which the majority of the purine residues are located on a single strand of the targeted duplex, resulting in CGC triplets across the three strands in the triplex.

Alternatively, the potential sequences that can be targeted for triple helix formation may be increased by creating a so called "switchback" nucleic acid molecule. Switchback molecules are synthesized in an alternating 5'-3', 3'-5' manner, such that they base pair with first one strand of a duplex and then the other, eliminating the necessity for a sizable stretch of either purines or pyrimidines to be present on one strand of a duplex.

Antisense RNA and DNA, ribozyme, and triple helix molecules of the invention may be prepared by any method known in the art for the synthesis of DNA and RNA molecules. These include techniques for chemically synthesizing oligodeoxyribonucleotides and oligoribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Moreover, various well known modifications to nucleic acid molecules may be introduced as a means of increasing intracellular stability and half-life. Possible modifications include but are not limited to the addition of flanking sequences of ribonucleotides or deoxyribonucleotides to the 5' and/or 3' ends of the molecule or the use of phosphorothioate or 2' O-methyl rather than phosphodiesterase linkages within the oligodeoxyribonucleotide backbone.

### VIII. Diagnostic & Prognostic Assays and Drug Screening Methods

The present invention provides method for determining whether a subject is at risk for developing a disease or condition characterized as an inflammatory bowel disease or disorder by detecting the disclosed biomarkers, i.e., the disclosed nucleic acid markers (see Table 1) and/or polypeptide markers for IBD encoded thereby.

In one embodiment, the subject method is used to diagnosis ischemic bowel diseases, and intestinal inflammations/allergies such as Coeliac disease, proctitis, eosnophilic gastroenteritis, mastocytosis, Crohn's disease and ulcerative colitis. With regard to inflammatory bowel disease, ulcerative colitis and Crohn's disease are characterized by infiltrative lesions of the bowel that contain activated neutrophils and macrophages.

In other embodiments, the subject method can be used to ascertain the degree of gut toxicity resulting from, e.g., a therapeutic or radiation regimen. Gut toxicity is a major limiting factor in radiation and chemotherapy treatment regimes. Pretreatment with KGF or other agents may have a cytoprotective effect on the small intestinal mucosa, allowing increased dosages of such therapies while reducing potential fatal side effects of gut toxicity. Monitoring the effectiveness of such protective therapeutics can be used to modulate the dosages.

In other embodiments, the subject method can be used as part of a diagnostic or prognostic kit for identifying risk of gastric ulcers or duodenal ulcers.

In clinical applications, human tissue samples can be screened for the presence and/or absence of the biomarkers identified herein. Such samples could consist of needle biopsy cores, surgical resection samples, bowel samples, lymph node tissue, or serum. For example, these methods include obtaining a biopsy, which is optionally fractionated by cryostat sectioning to enrich tumor cells to about 80% of the total cell population. In certain embodiments, nucleic acids extracted from these samples may be amplified using techniques well known in the art.

In one embodiment, the diagnostic method comprises determining whether a subject has an abnormal mRNA and/or protein level of the disclosed markers, such as by Northern blot analysis, reverse transcription-polymerase chain reaction (RT-PCR), *in situ* hybridization, immunoprecipitation, Western blot hybridization, or immunohistochemistry. According to the method, cells are obtained from a subject and the levels of the disclosed biomarkers, protein or mRNA level, is determined and compared to the level of these markers in a healthy subject. An abnormal level of the biomarker polypeptide or mRNA levels is likely to be indicative of IBD or risk of developing IBD.

Accordingly, in one aspect, the invention provides probes and primers that are specific to the unique nucleic acid markers disclosed herein. Accordingly, the nucleic acid probes comprise a nucleotide sequence at least 12 nucleotides in length, preferably at least 15 nucleotides, more preferably, 25 nucleotides, and most preferably at least 40 nucleotides, and up to all or nearly all of the coding sequence which is complementary to a portion of the coding sequence of a marker nucleic acid sequence, which nucleic acid sequence is represented in Table 1 or a sequence complementary thereto.

In one aspect, the method comprises in situ hybridization with a probe derived from a given marker nucleic acid sequence, which nucleic acid sequence is represented in Table 1 or a sequence complementary thereto. The method comprises contacting the labeled hybridization probe with a sample of a given type of tissue potentially containing IBD or pre-IBD cells as well as normal cells, and determining whether the probe labels some cells of the given tissue type to a degree significantly different (e.g., by at least a factor of two, or at least a factor of five, or at least a factor of twenty, or at least a factor of fifty) than the degree to which it labels other cells of the same tissue type.

Also within the invention is a method of determining the phenotype of a test cell from a given human tissue, e.g., whether the cell is (a) normal, or (b) IBD or pre-IBD, by contacting the mRNA of a test cell with a nucleic acid probe at least 12 nucleotides in length, preferably at least 15 nucleotides, more preferably at least 25 nucleotides, and most preferably at least 40 nucleotides, and up to all or nearly all of a sequence which is complementary to a portion of the coding sequence of a nucleic acid sequence represented in Table 1 or a sequence complementary thereto, and which is differentially expressed in tumor cells as compared to normal cells of the given tissue type; and determining the approximate amount of hybridization of the probe to the mRNA, an amount of hybridization either more or less than that seen with the mRNA of a normal cell.

Alternatively, the above diagnostic assays may be carried out using antibodies to detect the protein product encoded by the marker nucleic acid sequence, which nucleic acid sequence is represented in Table I or a sequence complementary thereto. Accordingly, in one embodiment, the assay would include contacting the proteins of the test cell or bodily fluid or fecal sample with one or more antibodies specific for gene products of a nucleic acid represented in Table 1 or a sequence complementary thereto, the marker nucleic acid being one which is expressed at a given control level in normal cells of the same tissue type as the test cell, and determining the approximate amount of immunocomplex formation by the antibody and the proteins of the test cell, wherein a statistically significant difference in the amount of the immunocomplex formed with the proteins of a test cell as compared to a normal cell of the same tissue type.

The subject invention further provides a method of determining whether a cell sample obtained from a subject possesses an abnormal amount of marker polypeptide which comprises (a) obtaining a cell sample from the subject, (b) quantitatively determining the amount of the marker polypeptide in the sample so obtained, and (c) comparing the amount of the marker polypeptide so determined with a known standard, so as to thereby determine whether the cell sample obtained from the subject possesses an abnormal amount of the marker polypeptide. Such marker polypeptides may be detected by immunohistochemical assays, dot-blot assays, ELISA and the like.

Immunoassays are commonly used to quantitate the levels of proteins in cell samples, and many other immunoassay techniques are known in the art. The invention is not limited to a particular assay procedure, and therefore is intended to include both homogeneous and heterogeneous procedures. Exemplary immunoassays which can be conducted according to the invention include fluorescence polarization immunoassay (FPIA), fluorescence immunoassay (FIA), enzyme immunoassay (EIA), nephelometric inhibition immunoassay (NIA), enzyme linked immunosorbent assay (ELISA), and radioimmunoassay (RIA). An indicator moiety, or label group, can be attached to the subject antibodies and is selected so as to meet the needs of various uses of the method which are often dictated by the availability of assay equipment and compatible immunoassay procedures. General techniques to be used in performing the various immunoassays noted above are known to those of ordinary skill in the art.

In another embodiment, the level of the encoded product, i.e., the product encoded by an IBD gene or a sequence complementary thereto, in a biological fluid (e.g., blood or urine) of a patient may be determined as a way of monitoring the level of expression of the marker nucleic acid sequence in cells of that patient. Such a method would include the steps of obtaining a sample of a biological fluid from the patient, contacting the sample (or proteins from the sample) with an antibody specific for a encoded marker polypeptide, and determining the amount of immune complex formation by the antibody, with the amount of immune complex formation being indicative of the level of the marker encoded product in the sample. This determination is particularly instructive when compared to the amount of immune complex formation by the same antibody in a control sample taken from a normal individual or in one or more samples previously or subsequently obtained from the same person.

As set out above, one aspect of the present invention relates to diagnostic assays for determining, in the context of cells isolated from a patient, if the level of a marker polypeptide is significantly reduced in the sample cells. The term "significantly reduced" refers to a cell phenotype wherein the cell possesses a reduced cellular amount of the marker polypeptide relative to a normal cell of similar tissue origin. For example, a cell may have less than about 50%, 25%, 10%, or 5% of the marker polypeptide that a normal control cell. In particular, the assay evaluates the level of marker polypeptide in the test cells, and, preferably, compares the measured level with marker polypeptide detected in at least one control cell, e.g., a normal cell and/or a transformed cell of known phenotype.

Of particular importance to the subject invention is the ability to quantitate the level of marker polypeptide as determined by the number of cells associated with a normal or abnormal marker polypeptide level. The number of cells with a particular marker polypeptide phenotype may then be correlated with patient prognosis. In one embodiment of the invention, the marker polypeptide phenotype of the lesion is determined as a percentage of cells in a biopsy which are found to have abnormally high/low levels of the marker polypeptide. Such expression may be detected by immunohistochemical assays, dot-blot assays, ELISA and the like.

Where tissue samples are employed, immunohistochemical staining may be used to determine the number of cells having the marker polypeptide phenotype. For such staining, a multiblock of tissue is taken from the biopsy or other tissue sample and subjected to proteolytic hydrolysis, employing such agents as protease K or pepsin. In certain embodiments, it may be desirable to isolate a nuclear fraction from the sample cells and detect the level of the marker polypeptide in the nuclear fraction.

The tissue samples are fixed by treatment with a reagent such as formalin, glutaraldehyde, methanol, or the like. The samples are then incubated with an antibody, preferably a monoclonal antibody, with binding specificity for the marker polypeptides. This antibody may be conjugated to a label for subsequent detection of binding. Samples are incubated for a time sufficient for formation of the immuno-complexes. Binding of the antibody is then detected by virtue of a label conjugated to this antibody. Where the antibody is unlabeled, a second labeled antibody may be employed, e.g., which is specific for the isotype of the anti-marker polypeptide antibody. Examples of labels which may be employed include radionuclides, fluorescers, chemiluminescers, enzymes and the like.

Where enzymes are employed, the substrate for the enzyme may be added to the samples to provide a colored or fluorescent product. Examples of suitable enzymes for use in conjugates include horseradish peroxidase, alkaline phosphatase, malate dehydrogenase and the like. Where not commercially available, such antibody-enzyme conjugates are readily produced by techniques known to those skilled in the art.

In one embodiment, the assay is performed as a dot blot assay. The dot blot assay finds particular application where tissue samples are employed as it allows determination of the average amount of the marker polypeptide associated with a single cell by correlating the amount of marker polypeptide in a cell-free extract produced from a predetermined number of cells.

In one embodiment, the present invention also provides a method wherein nucleic acid probes are immobilized on a DNA chip in an organized array. Oligonucleotides can be bound to a solid support by a variety of processes, including lithography. For example a chip can hold up to 250,000 oligonucleotides (GeneChip, Affymetrix). These nucleic acid probes comprise a nucleotide sequence at least about 12 nucleotides in length, preferably at least about 15 nucleotides, more preferably at least about 25 nucleotides, and most preferably at least about 40 nucleotides, and up to all or nearly all of a sequence which is complementary to a portion of the coding sequence of one or more marker nucleic acid sequence represented in Table 1.

The method includes obtaining a biopsy, which is optionally fractionated by cryostat sectioning to enrich tumor cells to about 80% of the total cell population. The DNA or RNA is then extracted, amplified, and analyzed with a DNA chip to determine the presence of absence of the marker nucleic acid sequences.

In one embodiment, the nucleic acid probes are spotted onto a substrate in a two-dimensional matrix or array. Samples of nucleic acids can be labeled and then hybridized to the probes. Double-stranded nucleic acids, comprising the labeled sample nucleic acids bound to probe nucleic acids, can be detected once the unbound portion of the sample is washed away.

The probe nucleic acids can be spotted on substrates including glass, nitrocellulose, etc. The probes can be bound to the substrate by either covalent bonds or by non-specific interactions, such as hydrophobic interactions. The sample nucleic acids can be labeled using radioactive labels, fluorophores, chromophores, etc.

Techniques for constructing arrays and methods of using these arrays are described in EP No. 0 799 897; PCT No. WO 97/29212; PCT No. WO 97/27317; EP No. 0 785 280; PCT No. WO 97/02357; U.S. Pat. No. 5,593,839; U.S. Pat. No. 5,578,832; EP No. 0 728 520; U.S. Pat. No. 5,599,695; EP No. 0 721 016; U.S. Pat. No. 5,556,752; PCT No. WO 95/22058; and U.S. Pat. No. 5,631,734.

In yet another embodiment, the invention contemplates using a panel of antibodies which are generated against the marker polypeptides of this invention, which polypeptides are encoded in Table 1. Such a panel of antibodies may be used as a reliable diagnostic probe for IBD. The assay of the present invention comprises contacting a biopsy sample containing cells, e.g., colon cells, with a panel of antibodies to one or more of the encoded products to determine the presence or absence of the marker polypeptides.

The diagnostic methods of the subject invention may also be employed as follow-up to treatment, e.g., quantitation of the level of marker polypeptides may be indicative of the effectiveness of current or previously employed IBD therapies as well as the effect of these therapies upon patient prognosis.

Accordingly, the present invention makes available diagnostic assays and reagents for detecting gain and/or loss of marker polypeptides from a cell in order to aid in the diagnosis and phenotyping of proliferative disorders arising from, for example, tumorigenic transformation of cells.

The diagnostic assays described above can be adapted to be used as prognostic assays, as well. Such an application takes advantage of the sensitivity of the assays of the invention to events which take place at characteristic stages in the progression of the disorder.

The methods of the invention can also be used to follow the clinical course of an IBD. For example, the assay of the invention can be applied to a tissue sample from a patient; following treatment of the patient for the IBD, another tissue sample is taken and the test repeated. Successful treatment will result in either removal of all cells which demonstrate differential expression characteristic of the IBD.

In yet another embodiment, the invention provides methods for determining whether a subject is at risk for developing a disease, such as a predisposition to develop IBD, for example UC or CD, associated with an aberrant activity of any one of the polypeptides encoded by nucleic acids of SEQ ID Nos: 1-146, wherein the aberrant activity of the polypeptide is characterized by detecting the presence or absence of a genetic lesion characterized by at least one of (i) an alteration affecting the integrity of a gene encoding a marker polypeptides, or (ii) the mis-expression of the encoding nucleic acid. To illustrate, such genetic lesions can be detected by ascertaining the existence of at least one of (i) a deletion of one or more nucleotides from the nucleic acid sequence, (ii) an addition of one or more nucleotides to the nucleic acid sequence, (iii) a substitution of one or more nucleotides of the nucleic acid sequence, (iv) a gross chromosomal rearrangement of the nucleic acid sequence, (v) a gross alteration in the level of a messenger RNA transcript of the nucleic acid sequence, (vii) aberrant modification of the nucleic acid sequence, such as of the methylation pattern of the genomic DNA, (vii) the presence of a non-wild type splicing pattern of a messenger RNA transcript of the gene, (viii) a non-wild type level of the marker polypeptide, (ix) allelic loss of the gene, and/or (x) inappropriate post-translational modification of the marker polypeptide.

The present invention provides assay techniques for detecting lesions in the encoding nucleic acid sequence. These methods include, but are not limited to, methods involving sequence analysis, Southern blot hybridization, restriction enzyme site mapping, and methods involving detection of absence of nucleotide pairing between the nucleic acid to be analyzed and a probe.

Specific diseases or disorders, e.g., genetic diseases or disorders, are associated with specific allelic variants of polymorphic regions of certain genes, which do not necessarily encode a mutated protein. Thus, the presence of a specific allelic variant of a polymorphic region of a gene in a subject can render the subject susceptible to developing a specific disease or disorder. Polymorphic regions in genes, can be identified, by determining the nucleotide sequence of genes in populations of individuals. If a polymorphic region is identified, then the link with a specific disease can be determined by studying specific populations of individuals, e.g, individuals which developed a specific disease, such as an IBD. A polymorphic region can be located in any region of a gene, e.g., exons, in coding or non coding regions of exons, introns, and promoter region.

In an exemplary embodiment, there is provided a nucleic acid composition comprising a nucleic acid probe including a region of nucleotide sequence which is capable of hybridizing to a sense or antisense sequence of a gene or naturally occurring mutants thereof, or 5' or 3' flanking sequences or intronic sequences naturally associated with the subject genes or naturally occurring mutants thereof. The nucleic acid of a cell is rendered accessible for hybridization, the probe is contacted with the nucleic acid of the sample, and the hybridization of the probe to the sample nucleic acid is detected. Such techniques can be used to detect lesions or allelic variants at either the genomic or mRNA level, including deletions, substitutions, etc., as well as to determine mRNA transcript levels.

A preferred detection method is allele specific hybridization using probes overlapping the mutation or polymorphic site and having about 5, 10, 20, 25, or 30 nucleotides around the mutation or polymorphic region. In a preferred embodiment of the invention, several probes capable of hybridizing specifically to allelic variants are attached to a solid phase support, e.g., a "chip". Mutation detection analysis using these chips comprising oligonucleotides, also termed "DNA probe arrays" is described e.g., in Cronin et al. (1996) Human Mutation 7:244. In one embodiment, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment.

In certain embodiments, detection of the lesion comprises utilizing the probe/primer in a polymerase chain reaction (PCR) (see, e.g. U.S. Patent Nos. 4,683,195 and 4,683,202), such as anchor PCR or RACE PCR, or, alternatively, in a ligase chain reaction (LCR) (see, e.g., Landegran et al. (1988) Science 241:1077-1080; and Nakazawa et al. (1994) PNAS 91:360-364), the latter of which can be particularly useful for detecting point mutations in the gene (see Abravaya et al. (1995) Nuc Acid Res 23:675-682). In a merely illustrative embodiment, the method includes the steps of (i) collecting a sample of cells from a patient, (ii) isolating nucleic acid (e.g., genomic, mRNA or both) from the cells of the sample, (iii) contacting the nucleic acid sample with one or more primers which specifically hybridize to a nucleic acid sequence under conditions such that hybridization and amplification of the nucleic acid (if present) occurs, and (iv) detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J.C. et al., 1990, Proc. Natl. Acad. Sci. USA 87:1874-1878), transcriptional amplification system (Kwoh, D.Y. et al., 1989, Proc. Natl. Acad. Sci. USA 86:1173-1177), Q-Beta Replicase (Lizardi, P.M. et al., 1988, Bio/Technology 6: 1197), or any other nucleic acid amplification method, followed by the detection of the amplified molecules using techniques well known to those of skill in the art. These detection schemes are especially useful for the detection of nucleic acid molecules if such molecules are present in very low numbers.

In a preferred embodiment of the subject assay, mutations in, or allelic variants, of a gene from a sample cell are identified by alterations in restriction enzyme cleavage patterns. For example, sample and control DNA is isolated, amplified (optionally), digested with one or more restriction endonucleases, and fragment length sizes are determined by gel electrophoresis. Moreover, the use of sequence specific ribozymes (see, for example, U.S. Patent No. 5,498,531) can be used to score for the presence of specific mutations by development or loss of a ribozyme cleavage site.

### IX: Drug Screening

Another aspect of the invention is directed to the identification of agents capable of modulating the growth state of an IBD cell. In this regard, the invention provides assays for determining compounds that modulate the expression of the marker nucleic acids (SEQ ID Nos: 1-146) and/or alter for example, inhibit the bioactivity of the encoded polypeptide.

Several in vivo methods can be used to identify compounds that modulate expression of the marker nucleic acids (e.g., an IBD gene) and/or alter for example, inhibit the bioactivity of the encoded polypeptide.

Drug screening is performed by adding a test compound to a sample of cells, and monitoring the effect. A parallel sample which does not receive the test compound is also monitored as a control. The treated and untreated cells are then compared by any suitable phenotypic criteria, including but not limited to microscopic analysis, viability testing, ability to replicate, histological examination, the level of a particular RNA or polypeptide associated with the cells, the level of enzymatic activity expressed by the cells or cell lysates, and the ability of the cells to interact with other cells or compounds. Differences between treated and untreated cells indicates effects attributable to the test compound.

Desirable effects of a test compound include an effect on any phenotype that was conferred by the IBD-associated marker nucleic acid sequence. Examples include a test compound that limits the overabundance of mRNA, limits production of the encoded protein, or limits the functional effect of the protein. The effect of the test compound would be apparent when comparing results between treated and untreated cells.

### X. Transgenic Animals

Another aspect of the present invention relates to transgenic non-human animals having germline and/or somatic cells in which the biological activity of one or more IBD genes are altered by a chromosomally incorporated transgene. Such animals can be used as models for inflammatory bowel diseases or disorders, e.g., for understanding the pathology of disease and/or drug screening.

In one embodiment, the present invention provides a desired non-human animal or an animal (including human) cell which contains a predefined, specific and desired alteration rendering the non-human animal or animal cell predisposed to and inflammatory bowel disease.

In embodiments where the IBD gene is down-regulated in the disease state, the transgene may encode a mutant protein, such as dominant negative protein which antagonizes at least a portion of the biological function of a wild-type protein. Yet in other embodiments, the transgene can encode an antisense transcript which, when transcribed from the transgene, hybridizes with a gene or a mRNA transcript thereof, and inhibits expression of the gene. In still other embodiments, the transgene can, by such mechanisms as homologous recombination, knock-out the endogenous IBD gene.

A preferred transgenic non-human animal of the present invention has germline and/or somatic cells in which one or more alleles of a gene are disrupted by a chromosomally incorporated transgene, wherein the transgene includes a marker sequence providing a detectable signal for identifying the presence of the transgene in cells of the transgenic animal, and replaces at least a portion of the gene or is inserted into the gene or disrupts expression of a wild-type protein.

In embodiments where the IBD gene is up-regulated in the disease state, the transgene may encode a wild-type IBD gene product, and the transcriptionally regualtory sequences of the transgene can be used to cause overexpression of the IBD gene. Likewise, mutant IBD genes can be used which encode IBD proteins that are consitutitively or regulatively activated to mimic overexpression of the endogenous IBD gene.

Furthermore, it is contemplated that cells of the transgenic animals of the present invention can include other transgenes, e.g., which alter the biological activity of a second tumor suppressor gene or an oncogene. For instance, the second transgene can functionally disrupt the biological activity of a tumor suppressor gene, such as p53, p73, DCC, p21^{cip1} p27^{kip1} Rb, Mad or E2F. Alternatively, the second transgene can cause overexpression or loss of regulation of an oncogene, such as ras, myc, a cdc25 phosphatase, Bcl-2, Bcl-6, a transforming growth factor, neu, int-3, polyoma virus middle T antigen, SV40 large T antigen, a papillomaviral E6 protein, a papillomaviral E7 protein, CDK4, or cyclin D1.

Still another aspect of the present invention relates to methods for generating non-human animals and stem cells having a functionally disrupted endogenous gene. In a preferred embodiment, the method comprises the steps of:
(i) constructing a transgene construct including (a) a recombination region having at least a portion of an IBD gene, which recombination region directs recombination of the transgene with the gene, and (b) a marker sequence which provides a detectable signal for identifying the presence of the transgene in a cell;
(ii) transfering the transgene into stem cells of a non-human animal;
(iii) selecting stem cells having a correctly targeted homologous recombination between the transgene and the gene;
(iv) transfering cells identified in step (iii) into a non-human blastocyst and implanting the resulting chimeric blastocyst into a non-human female; and
(v) collecting offspring harboring an endogenous gene allele having the correctly targeted recombination.

Yet another aspect of the invention provides a method for evaluating the potential of an agent to cause an IBD or to protect against development of an IBD by (i) contacting a transgenic animal of the present invention with a test agent, and (ii) ascertaining the presence, and more preferably the level, of onset or degree of severity of an inflammatory bowel disease or disorder, and comparing that with an untreated transgenic animal or transgenic animal treated with a control agent.

The following Table 1 teaches genes whose up-regulation or down-regulation, as indicated by "↑" and "↓", respectively, has been found to be associated with UC and CD. The genes are grouped according to their general functionality.

### X Exemplification

| | **UC** | **CD** | **Accession No.** | **Gene Names** | **Chromosome** | **Microsatellite Markers** |
|---|---|---|---|---|---|---|
| I | ↑21.4 | ↑12.8 | Y00787 | MDNCF/IL-8 | 4q13-q21 | D4S392-D4S2947 |
| I | ↑15.3 | | X54489 | MGSA(GROI) | 4q21 | D4S400-D4S1534 |
| I | ↑7.9 | | M57731 | MIP-2a (GR02) | 4q21 | D4S392-D4S2947 |
| I | ↑8.9 | ↑4.1 | M28130 | IL8 | 4q13-q21 | D4S392-D4S2947 |
| I | ↑6.8 | ↑3.9 | X57351 | IP-10 | 11 | pTEL-D11S1318 |
| I | ↑6 | | J04130 | MIP-1(1β/SCYA4 | 17q21 | D17S933-Dl7S800 |
| I | ↑3.4 | | X53800 | MIP-2β (GR03) | 4q21 | D4S400-D4S1534 |
| I | ↑3.2 | | M69203 | MIP-1β(3/SCYA2 | 17q21 | D175933-D17S800 |
| I | ↑4.6 | | X04500 | pro-IL-1β | 2q14 | D2S293-D2S121 |
| I | ↑3.5 | | X53296 | IL-IRA | 2ql4 | D2S293-D2S121 |
| I | ↑3.3 | | X04602 | 1L-6 | 7q21 | D7S829-D7S673 |
| I | ↑3 | | J03756 | Growth hormone2 2 (G112) | 17q22-q24 | D17S794-Dl78795 |
| I | ↓-3.5 | | D16431 | Hepatoma-derived growth factor (HDGF) | 17q2-q24 | D17S794-Dl78795 |
| II | 35.5 | | S75256 | Neutrophil lipocalin (HNL) | - | - |
| II | ↑10.4 | | X99133 | Neutrophil gelatinase-associated lipocalin (NGAL) | 9q34 | D9S1821-D9S159 |
| II | ↑8.7 | | X85781 | Nitric oxide synthase (NOS2) | ? | - |
| II | ↑5.1 | | X65965 | Mitochondrial superoxide dismutase (SOD2) | 6q25 3 | D6S442-D6S1581 |
| II | ↑5.5 | ↑4.6 | M22430 | Phospholipase A2, group IIA (PLA2G2A) | 1p35 | - |
| II | ↑5.3 | | X51441 | Serum amyloid A (SAA) | 11p | - |
| II | ↑3.9 | | J03474 | Serum amyloid A (SAA1) | 11p15.1 | D11S921-D11S1369 |
| II | ↑3.7 | | M21119 | Lysozyme | | |
| II | ↑3.4 | | D00408 | Cytochrome P450 IIIA, polypeptide 7 (CPY3A7) | 7 | D7S479-D7S2545 |
| II | ↓4.2 | | D14662 | Anti-oxidant protein 2 | 1 | D1S2790-D1S2640 |
| II | ↓4.4 | | X64177 | Metallothionein | - | - |
| II | ↓-8 | | J03910 | Metallothionein-1G (MT1G) | 16q13 | D16S3057-D16S514 |
| III | ↑155 | ↑17.8 | L08010 | Regenerating islet-derived 1β (REG1B) | 2p12 | D2S286-D2S169 |
| III | ↑75 | ↑36.4 | J05412 | Regenerating islet-derived 1 α (REG1A) | 2p12 | D2S139-D2S289 |
| III | ↑9.7 | ↑10.2 | L15533 | Pancreatits-associated protein (PAP) | 2p12 | D2S169-D2S139 |
| III | ↑58.8 | | IIG35b6-HT3769 | Zinc Finger Protein. | - | - |
| III | ↑55.1 | ↑12.5 | M87789 | Ig γ 3 (1GHG3) | 14q32.33 | D14S65-qTEL |
| III | ↑17.5 | ↑4.7 | M26311 | S100A9/calgranulin B | 1q12-q22 | D1S514-D1S2635 |
| III | ↑10.8 | ↑3.6 | U08021 | Nicotinamide N-methyltransferase (NNMT) | 11q23.1 | D(151347-D11S939 |
| III | ↑5 | | M72885 | GOS2 | - | - |
| III | ↑3.9 | ↑4.2 | X65614 | S100 calcium-binding protein (S100P) | 4p16 | - |
| III | ↑3.9 | | U01691 | Annexin AV (ANXA5) | 4q28-q32 | D4S2945-D4S430 |
| III | ↑3.7 | | U22431 | Hypoxia-inducible factor 1a(H1F1A) | 14q21-q24 | D14S1038-D14S290 |
| III | ↑3.2 | | HG3494-HT3688 | NF-116 | - | - |
| III | | ↑3.3 | X99585 | Suppressor of mif two 3 (SMT3H2) | 8 | D8S257-D8S508 |
| III | | ↑3.1 | U66617 | SW1/SNF related regulator of chromatin(SMARCDI) | 12q13-q14 | D12S333-D12S325 |
| III | | ↑3.2 | L19067 | NF-kappa-B p65 subunit | - | - |
| III | ↓-3.1 | ↓-3.2 | D14520 | Basic transcription element binding proteins (2BTEB2) | - | - |
| III | | ↓-3.2 | M21142 | Guanine nucleotide-binding protein α (GNAS 1 ) | 20q13.2-q 13.3 | D20S183-D20S173 |
| III | | ↓-4.9 | AD000684 | Liver specific bHLH-zip | - | - |
| III | ↓-3.1 | | S37730 | Insulin-like growth factor binding protein 2 (IGFBP2) | 2q33-q34 | D2S137-D2S164 |
| III | ↓-3.8 | | L11672 | Zinc finger protein 91 (ZNF91) | 19p13.1-p12 | - |
| III | ↓-3.8 | | D32257 | Transcription factor IIIa | 3q12.3-q13.1 | D13S221-D13S1244 |
| III | ↓-5.5 | ↓-3.3 | M32886 | Sorcin (SRI) | 7q21.1 | D7S524-D7S657 |
| III | ↓-12.5 | ↓-5.9 | M16364 | Creatine kinase, brain (CKB) | 14q32 | D14S65-qTEL |
| IV | ↑4.8 | | U21049 | Epitheial protein upregulated in carcinoma (DD96) | - | - |
| IV | ↑3.5 | | D38583 | Calgizzarin (S100A11) | 7, 17, 4 | D7S529-D7S4 84, D717_{S}1352-D17S785 |
| IV | | | | | | D4S1615-D4S1579 |
| IV | | ↑3.2 | L42176 | Downregulated in rhabdomyosarcoma (DRAL) | 2q12-q14 | D2S113-D2S176 |
| IV | ↓-3.5 | | L07648 | Max-interacting protein 1 (MXII) | 10q24-q25 | D10S597-D10S1681 |
| IV | ↓-4.4 | | L02785 | Down regulated in adenoma (DRA) | 7q31 | D7S2420-D7S523 |
| V | 9.2 | | M57466 | HLA-DPB1 | 6p21.3 | D6S1558-D6S1616 |
| V | ↑5.9 | | HG3576-HT3779 | MHC IIβ W52 | - | - |
| V | ↑5 | | HG1872-HT1907 | MHC Dg | - | - |
| V | ↑4.9 | | M33600 | HLA-DRB1 | 6p21. 3 | D6S1558-D6S1616 |
| V | ↑4.1 | | X00274 | HLA-DR α heavy chain | - | - |
| V | ↑4 | | X62744 | HLA-DMA | 6p21 .3 | D6S1S58-D6S1616 |
| V | ↑4 | | M16276 | MHC II HLA-DR2-Dw12 DQw1-β | - | - |
| V | ↑3.4 | | X03068 | HLA-D II antigen DQw1.1 β | - | - |
| V | ↑10.8 | | X57809 | 1g λ gene cluster (IGL@) | 22q11.1-q11.2 | D22S420-D22S1144 |
| V | ↑9 | ↑3 | L23566 | Ig heavy chain, VDJRC | - | - |
| V | ↑8.6 | | L02326 | Ig λ-like polypeptide 2 (IGLL2) | 22q11.2 | D22S1144-D22S280 |
| V | ↑6.8 | | M63438 | Ig rearranged γ chain, V-J-C region | - | - |
| V | ↑5.6 | | X72475 | Rearranged Ig κ light chain | - | - |
| V | ↑4.6 | | M13560 | 1a-associated invariant γ-chain (CD74) | - | - |
| V | ↑4.1 | | M34516 | O light chain protein | - | - |
| | | | | 14 1 | | |
| V | ↑4 | | X73079 | Polymeric lg receptor | - | - |
| V | ↑3.7 | | S71043 | Ig alpha 2 - IgA heavy chain allotype 2 | - | - |
| V | ↑3.7 | | X00437 | T-cell specific proteir/T-cell receptor | - | - |
| V | ↑5.9 | | J03909 | Interferon γ-inducible protein 30 (IF130) | 19p13 1 | D19S899-D19S407 |
| V | ↑3 | | M63838 | Interferon γ-inducible protein (IFI16) | - | - |
| V | | ↑4.8 | D28915 | Microtubular aggregate protein p44 | 1 | D1S203-D152865 |
| | ↓-4.2 | ↓-3.4 | M13755 | Inteferon stimulated protein 15-kDa (1SG15) | 1 | D1S243-D1S468 |
| | | ↓-3.4 | D11086 | IL-2 receptor y chain (IL2RG) | Xq13.1 | DXS983-DXS995 |
| V | ↓-3 | | M84526 | Complement factor D (DF) | - | pTEL-D 19S413 |
| V | ↓-3.9 | | M38690 | CD9 antigen | 12p13 | D12S99-D12S358 |
| Vl | ↑20.4 | ↑40.8 | M97925 | Defensin 5 (DEFA5) | 8pter-p21 | D8S552-D8S549 |
| VI | ↑6.8 | ↑7.7 | U33317 | Defensin 6 (DEFA6) | 8pter-p21 | D8S277-D8S550 |
| VII | ↑16.2 | ↑3.3 | L23808 | MMP-12 (Macrophage elastase) | 11q22.2-q22.3 | D11S1339-D11S1343 |
| VII | ↑6.4 | | J05070 | MMP-9 (GelatinaseB) | 20q11.2-q13.1 | D20S119-D20S197 |
| VII | ↑4.7 | | X54925 | MMP-1 (Interstitial collagenase) | 11q22.3 | D11S1339-D11S1343 |
| VII | ↑4.2 | | X05232 | MMP-3 (Stromelysin 1) | 11q22.3 | D11S1339-D11S1343 |
| VII | ↑13.3 | ↑3.8 | L10343 | Elastase specific inhibitor (Elafin) | 20q12-q13 | D205119-D20S197 |
| VII | ↑11 | ↑3.1 | Z74616 | COL1A2 | 2q37 | D2S2158-D2S125 |
| VII | ↑7.3 | | X52022 | COL6A3 | 2q37 | D2S2158-D2S125 |
| VII | ↑6.9 | ↑3.6 | M55998 | COL-1A1 | 17q21.3-q22 | D17S791-D17S794 |
| VII | ↑4.8 | | X06700 | COL3A1 | 2q31 | D2S2257-D2S115 |
| VII | ↑4.7 | | X15882 | COL6A2 | 21q22.3 | - |
| | ↑3.9 | | X05610 | COL4A2 | 13q34 | D13S285-qTEL |
| VII | ↑3.7 | ↑3.3 | HG2157-HT2227 | Mucin 4 (MUC4) | 3q29 | - |
| VII | ↑3.1 | | X52003 | Trefoil factor 1 (TFF1) | 21 q22 3 | D21S1259-qTEL |
| VII | | ↑4.6 | M22406 | Intestinal mucin | - | - |
| VII | ↑6.4 | | J03040 | Osteonectin (SPARC) | 5q3l.3-q32 | D5S436-D5S470 |
| VII | ↑4 | ↑3.2 | X17042 | Proteoglycan 1 (PRG1) | 10q22. 1 | D10S210-D10S537 |
| VII | ↑3.9 | | D11428 | Penpheral myelin protein 22 (PMP22) | 17p12-p11.2 | D17S804-D17S799 |
| VII | ↑3.8 | | X02761 | Fibronectin I (FN1) | 2q34 | D2S137-D2S164 |
| VII | ↑3.7 | | M77349 | Transforming growth factor beta-induced (TGFfβI) | 5q31 | D5S393-D5S500 |
| VII | ↑3.2 | | D13666 | Osteoblast specific factor 2 (OSF-2) | 13 | D138267-D13S1253 |
| VII | ↑3.1 | | M10321 | von Willebrand factor | 12p13.3 | D12S99-D12S358 |
| VII | ↑3 | | L09190 | Trichohyalin (THH) | 1q21-q23 | D1S439-D1S459 |
| VII | | ↑3.1 | D88422 | Cystatin A (CSTA) | 3q21 | - |
| VII | | ↑4.7 | X58199 | Adducin 2 (ADD2) | 2p13-p14 | - |
| VII | | ↑3.7 | M86933 | Amelogenin (AMELY) | Yp11.2 | - |
| VII | | ↓-3.2 | D45370 | Adipose specific collagen-like 2 (APM2) | 10 | D10S1786-D10S541 |
| VII | | ↓-3.8 | X73501 | Cytokeratin 20 | - | - |
| VIII | ↑50.5 | | D28416 | Esterase D (ESD) | 13q14.1-q14.2 | D13S328-D13S168 |
| VIII | ↑4.7 | | M15656 | Aldolase B | 9q21.3-q22.2 | D15S202-D15S157 |
| VIII | | ↑6.3 | J04040 | Glucagon (GCG) | 2q36-q37 | D2S156-D2S376 |
| VIII | | ↓-4.4 | L31801 | Monocarboxylate | 1p13.2-p12 | D1S418-D1S514 |
| | | | | transporter 1 (MCTI) | | |
| VIII | ↓-3 | | D10523 | Oxoglutarate dehydrogenase (OGDH) | 7p14-p13 | D7S521-D7S478 |
| VIII | ↓-4 | | M12963 | Alcohol dehydrogenase 1a (ADH 1 ) | 4q21-q23 | - |
| VIII | ↓-4 5 | | Y00339 | Carbonic anhydrase II (CA2) | 8q22 | D8S275-D8S273 |
| VIII | ↓-4.9 | ↓-3.1 | L10955 | Carbonic anhydrase IV (CA4) | 17q23 | - |
| VIII | ↓-12.7 | ↓3.1 | L05144 | Phophoenolpyruvate carboxykinase 1,soluble (PCK1 ) | 20q13.31 | D20S183-D20S173 |
| VIII | ↑3 | | U07158 | Syntaxin 4A (STX4A) | - | - |
| VIII | | ↑3 | L27706 | Chaperonin subunit 6A (CCT6A) | 7 | D7S530-D7S509 |
| VIII | | ↓-3.1 | J04093 | UDP-gluycosyltransferase 1 (UGT1) | 2 | D2S2159-D2S125 |
| V1II | ↓-3.2 | | U20499 | Sulfotransferase family 1A (SULT1A3) | 16p11.2 | - |
| VIII | ↑3 | | M15182 | P-glucuronidase (GUSB) | 7q21 11 | - |
| VIII | ↑4 | | U08854 | UDP glucuronosyltransferase precursor (UGT2B15) | 4q13 | D4S1619-D4S392 |
| VIII | ↑5 | | D87292 | Thiosulfate sulfurtransferase (TST) | 22 | D22S277-D22S283 |
| VIII | ↑13 | ↑4 | M22324 | Aminopeptidase N/CD13 (ANPEP) | I5q25-q26 | D15S202-D15S157 |
| VIII | | ↑7 | M22960 | Protective protein for b-galactosidase (PPGB) | 20q13,1 | D20S119-D20S197 |
| VIII | ↑3.4 | | X90908 | Fatty acid binding protein 6 (FABP6) | 5q23-q35 | - |
| VIII | | ↑4.1 | J02874 | Fatty acid binding protein 4 (FABP4) | 8q21 | - |
| VIII | ↑3 | | M10050 | Fatty acid binding protein 1 (FABP1) | 11pl5.5 | D11S1318-D11S909 |
| VIII | ↑3 | | L24774 | Mitochondrial d3, d2-CoA-isomerase | - | |
| VIII | ↑4 | | D16294 | Mitochondrial 3-oxoacyl-CoA thiolase (ACAA2) | 18 | D18SU1118-D18S474 |
| VIII | ↑4 | | M77144 | 3 b-hydroxysteroid dehydrogenase (HSD3B2)) | 1p13.1 | D1S418-D1S514 |
| VIII | ↑5 | | D10511 | Mitochondrial acetoacetyl-CoA thiolase | - | - |
| VIII | ↑7 | | Z80345 | Acyl-Coenzyme A dehydrogenase (ACADS) | 12q22-qter | D12S366-D12S340 |
| VIII | ↑7 | | L11708 | 17 b-hydroxysteroid dehydrogenase II (HSD17B2) | 16q24.1-q24.2 | D16S515-D16S422 |
| VIII | ↑7 | | U26726 | 11 b-hydroxysteroid dehydrogenase II (HSD11B2) | 16q22 | D16S3031-D16S3139 |
| VIII | ↓3.5 | | X93036 | MAT8 protein | 19 | D19S425-D19S418 |
| VIII | ↓-12.2 | ↓-14 | M97496 | Guanylate cyclase activator 1B (UCAIB) | 6p21.1 | D1S2843-D1S417 |
| VIII | | ↑4.2 | D17400 | 6-pyruvoyl-tetrahydropterin synthase (PCBD) | 10q22 | D10S210-D10S537 |
| VIII | | ↑3.3 | D21262 | KIAA0035 | - | - |
| VIII | | ↑3.1 | AB002365 | KIAA0367 | - | - |
| VIII | | ↓-4.5 | M11119 | Endogenous retrovirus | - | - |
| | | | | envelope region | | |
| VIII | ↓-3.1 | | M19961 | Mitochondrial cytochrome c oxidase Vb (COX5B) | 2cen-q13 | D2S113-D2S176 |
| VIII | ↓-3.1 | | D26129 | Pancreatic ribonuclease (RNASE1) | 14 | pTEL-D14S293 |
| VIII | ↓-3.1 | | U77643 | K12 (SECTM1) | 17q25 | - |
| VIII | ↓-4 | | | HG3991-HT4261 | Cpg-Enriched DNA, clone E18 | |
| VIII | | | | | | |

| | | | | | | |
|---|---|---|---|---|---|---|
| **I** Chemokines + cytokines and growth factors **II** Inflammatory mediators **III** Cell cycle regulators/ transcription factors **IV** Caner Related **V** HLA or immune function genes **VI** Antimicrobial **VII** ECM and remodelling **VIII** Other Carbohydrate metabolism Fatty acid metabolism Protein folding/modification/degradation | | | | | | |

### AII. Equivalents

Those skilled in the art will recognize, or be able to ascertain, using not more than routine experimentation, many equivalents to the specific embodiments of the invention described herein. Such specific embodiments and equivalents are intended to be encompassed by the following claims.

All patents, published patent applications, and publications cited herein are incorporated by reference as if set forth fully herein.

The invention is further characterised by the following non-limiting embodiments:
1. A method for identifying genes which are up-or down-regulated in intestinal tissue of patients who have, or are at risk of developing, an inflammatory bowel disease or disorder, comprising:
   (i) generating a first library of nucleic acid probes representative of genes expressed by intestinal tissue of an animal without apparent symptoms and/or risk for an inflammatory bowel disease or disorder;
   (ii) generating a second library of nucleic acid probes representative of genes expressed by intestinal tissue of an animal which has symptoms of, and/or is at risk for developing, an inflammatory bowel disease or disorder; and
   (iii) identifying genes that up-or down-regulated, e. g., by at least a predetermined fold difference, in the second library of nucleic acids relative to the first library of nucleic acids.
2. The method of embodiment 1, including the further step of cloning those genes which are up-or down-regulated.
3. The method of embodiment 1, including the further step of generating nucleic acid probes for detecting the level of expression of those genes which are up-or down-regulated.
4. The method of embodiment 1, including the further step of providing kits, such as microarrays, including probes for detecting the level of expression of those genes which are up-or down-regulated.
5. A method for determining the phenotype of a cell, particularly a cell of intestinal origin, comprising detecting the differential expression, relative to a normal cell, of at least one gene shown in Table 1 (herein the"IBD gene set"), or other IBD genes identified according to the method of embodiment 1.
6. The method of embodiment 5, wherein the assay detects a difference in the level of expression of an IBD gene of at least a factor of two.
7. The method of embodiment 5, which is used to assess a patient's risk of having, or developing, an inflammatory bowel disease.
8. A kit for assessing a patient's risk of having or developing an inflammatory bowel disease, comprising (i) detection means for detecting the differential expression, relative to a normal cell, of at least five genes shown in Table 1 (herein the"IBD gene set") or the gene products thereof; and (ii) instructions for correlating the differential expression of IBD genes or gene products, if any, with a patient's risk of having or developing an inflammatory bowel disease.
9. The kit of embodiment 8, wherein the detection means includes nucleic acid probes for detecting the level of mRNA of the IBD genes.
10. The kit of embodiment 8, wherein the detection means includes nucleic acid probes for detecting the presence of mutations or changes in methylation patterns to genomic sequences encoding the IBD genes.
11. The kit of embodiment 8, wherein the detection means includes an immunoassay for detecting the level of IBD gene products.
12. A method of doing a business for assessing a patient's risk of having or developing an inflammatory bowel disease, comprising
   (i) providing a service for determining the level of expression of an IBD gene set or gene products thereof, and comparing the level of expression to a normal cell; and
   (ii) assessing a patient's risk of having or developing an inflammatory bowel disease, if any, by determining the correlation between the differential expression of IBD genes or gene products with known changes in expression of IBD genes measured in other patients suffering from an inflammatory bowel disease.
13. A method for treating a patient who has developed, or is at risk of developing, an inflammatory bowel disease comprising: (i) detecting the differential expression, relative to a normal cell, of at least one IBD gene; (ii) proscribing a course of treatment dependent on the level of expression of the IBD gene (s) relative to normal cells.
14. A nucleic acid array comprising a solid support and displayed thereon nucleic acid probes which selectively hybridize to at least **25** different IBD genes.
15. The array of embodiment **14**, wherein the solid support is selected from the group consisting of paper, membranes, filters, chips, pins, and glass.
16. A drug screening assay comprising (i) administering a test compound to an animal having an inflammatory bowel disease, or a cell composition isolated therefrom; (ii) comparing the level of IBD gene expression in the presence of the test compound with one or both of the level of IBD gene expression in the absence of the test compound or in normal cells; wherein test compounds which cause the level of expression of one or more IBD genes to approach normal are candidates for drugs to treat inflammatory bowel diseases.
17. A method for treating an animal having an inflammatory bowel disease comprising administering a compound identified by the assay of embodiment **16**.
18. A pharmaceutical preparation for treating an animal having an inflammatory bowel disease comprising a compound identified by the assay of embodiment 16 and a pharmaceutically acceptable excipient.

## Claims

1. A method for determining whether a cell of intestinal origin has an inflammatory bowel disease (IBD) phenotype, comprising detecting the differential expression, relative to a normal colon cell, of
(i) at least one gene shown in Table 1, wherein the at least one gene is the Co16A3 gene, or
(ii) at least one of the genes in group VII of the IBD gene set shown in Table 1.

2. The method of claim 1, wherein the method detects a difference in the level of expression of the IBD gene of at least a factor of two.

3. The method of claim 2, wherein the method is used to assess a patient's risk of having, or developing, an inflammatory bowel disease.

4. The method of claim 1, wherein the IBD is ulcerative colitis (UC).

5. The method of claim 1, wherein the IBD is Crohn's disease (CD).

6. The method of claim 1, wherein the cell is obtained from a needle biopsy core, a surgical resection sample, a bowel sample, lymph node tissue, or serum.

7. The method of claim 1, wherein the method detects a difference in the level of expression of each of the IBD genes using Northern blot analysis, reverse transcription-polymerase chain reaction, in situ hybridization, or a library.

8. The method of claim 5, wherein the library comprises:
(a) nucleic acid probes that are complementary to the IBD genes; and
(b) a substrate to which the nucleic acid probes are bound.

9. The method of claim 6, wherein the substrate is selected from the group consisting of paper, membranes, filters, chips, pins, and glass.

10. The method of claim 6, wherein the nucleic acid probes are bound to the substrate by covalent bonds or hydrophobic interactions.

11. A kit for assessing a patient's risk of having or developing an inflammatory bowel disease, comprising
(a) detection means for detecting the differential expression, relative to a normal cell, of
(i) at least one gene shown in Table 1, wherein the at least one gene is the Co16A3 gene, or
(ii) at least one of the genes in group V of the IBD gene set shown in Table 1; or the gene products thereof; and
(b) instructions for correlating the differential expression of IBD genes or gene products, if any, with a patient's risk of having or developing an inflammatory bowel disease.

12. The kit of claim 6, wherein the detection means includes nucleic acid probes for detecting the level of mRNA of the IBD genes.

13. The kit of claim 6, wherein the detection means includes nucleic acid probes for detecting the presence of mutations or changes in methylation patterns to genomic sequences encoding the IBD genes.

14. The kit of claim 6, wherein the detection means includes an immunoassay for detecting the level of IBD gene products.

15. At least.one gene shown in Table 1, wherein the at least one gene is the Co16A3 gene; or at least one of the genes in group VII of the IBD gene set shown in Table 1, for use in a method for treating a patient who has developed, or is at risk of developing, an inflammatory bowel disease wherein the treatment comprises:
(i) detecting the differential expression, relative to a normal cell, of at least one IBD gene;
(ii) (ii) proscribing a course of treatment dependent on the level of expression of the IBD gene (s) relative to normal cells.
